# EUROPEAN PATENT APPLICATION

(11) **EP 3 885 440 A1**
(43) Date of publication of application: **29.09.2021**
(21) Application number: 20382236.6
(22) Date of filing: 26.03.2020
(51) Int. Cl.: C12N 15/861

(54) **SPLIT INTEINS AND THEIR USES**

(71) Applicant: SPLICEBIO, S.L., 08028 Barcelona (ES)
(72) Inventor: FRUTOS DOMÍNGEZ, Silvia, 08028 Barcelona (ES); CAELLES VIDAL, Gerard, 08028 Barcelona (ES); OTERO BILBAO, Anabel, 08028 Barcelona (ES); VILA PERELLÓ, Miquel, 08028 Barcelona (ES)
(74) Representative: Hoffmann Eitle

(57) **Abstract**

The present invention relates to methods of use of engineered split inteins, as well as their combination with degradation signals (destabilizing domains, degrons) to reconstitute large genes for gene therapy.

## Description

### Technical field of the invention

The present invention is comprised within the field of biotechnology, it specifically relates to split inteins and their uses.

### Background of the invention

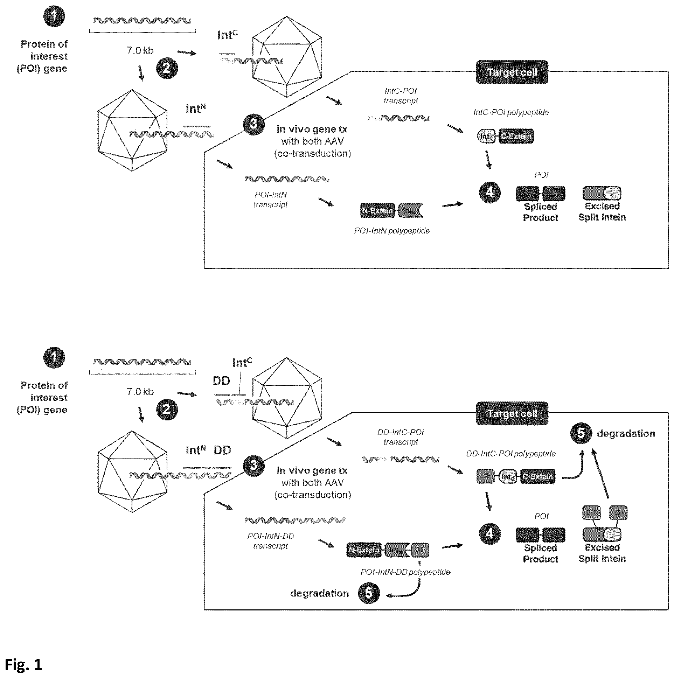

It is known that genes that cannot be encapsulated inside an AAV (adeno-associated virus) for gene therapy, can be split in two fragments. Each fragment should be thus fused to a split intein and delivered in their own AAV. Upon infection of the target cells inteins will enable the generation of the desired target protein (see figure 1).

Nevertheless, one of the key limitations of the approach is the accumulation of protein intermediates (N-Extein-ItnN and IntC-C-Extein). as well as the excised split intein. To address this problem, in the instant application, we have shown that certain inteins can be combined with certain degradation signals providing a significant reduction in the accumulation of such protein intermediates. The instant invention focuses on this combination of inteins and degradation signals. We have also identified certain intein-degron combinations that contribute to increase splicing yields in vivo.

### Brief description of the figures

**Figure 1****.** General scheme of the strategies to use inteins, and inteins combined with degrons to reconstitute large proteins for gene therapy. Top panel: (1) the gene of interest is recombinantly split in a judiciously selected position and the resulting 5' end is recombinantly fused to and IntN, and the 3' to the IntC, in such a way that upon protein expression the N-terminal fragment of the protein will be expressed with the IntN fused to its C-terminus, and the C-terminal fragment of the protein will be expressed with the IntC fused to its N-terminus. (2) Each of the construct is encapsulated in a separate AAV. These two AAVs are administered to a patient, so that they are co-transduced to certain cells (3). Upon co-transduction the DNA delivered via the AAVs will be transcribed into RNA and translated into proteins, and at the protein level the inteins will perform the protein trans-splicing reaction to reconstitute the desired splided product (4). Bottom planel: inteins are combined with degradation signals to prevent accumulation of the starting materials, eliminate the excised inteins. Appropriate selection of degradation signals also allows to increase the amount of spliced product. Degrons are fused to the C-terminus of the N-intein and the N-terminus of the C-intein.

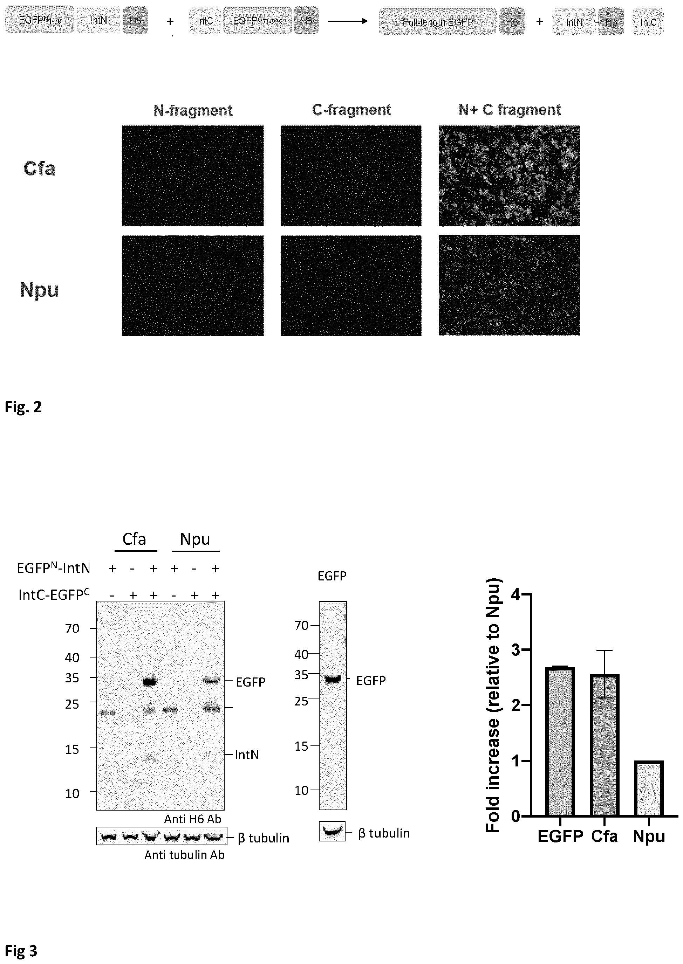

**Figure 2****.** Top panel: reaction scheme between the EGFP^{N}-IntN-H6 and IntC-EGFP^{C}-H6 constructs, which results in the formation of full length EGFP with a H6 tag, and excision of the inteins. Lower panel: flourescence microscopy images of cells transfected with the N-, C-terminal fragments or co-transfected with both fragments, using either the Cfa or the Npu intein.
**Figure 3****.** Western blot analysis of lysed HEK293 cells transfected with EGFP-intein polynucleotides. Left panel: western blot analysis of cells transfected with EGFP-intein plasmids. Cells were transfected with a full length EGFP plasmid, or either a EGFP^{N}-IntN, an IntC-EGFP^{C} plasmid, or co-transfected with both. Plasmids containing the Cfa or the Npu intein were tested. Right panel: quantificaton of fold increase of spliced product, relative to Npu. Product yield was determined by densitometry, using β-tubulin as a loading control. The graph represents the fold increase of EGFP product, relative to Npu, when cells were transfected with either full lenth EGFP plasmids (EGFP), co-transfected with EGFP^{N}-CfaN and CfaC-EGFP^{C} (Cfa), or with EGFP^{N}-NpuN and NpuC-EGFP^{C} (Npu).

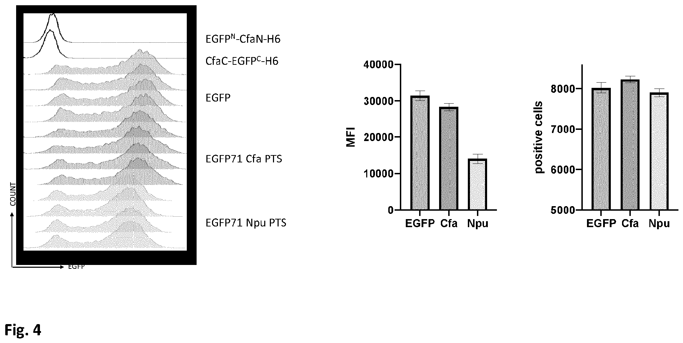

**Figure 4****.** HEK293 transfected cells were analyzed by flow cytometry. Left panel: flow cytometry data of cells transfected with different constructs or combinations of constructs. Black curves correspond to controls transfected with either an N-terminal fragment or C-terminal fragment of EGFP. In blue are 4 replicates of cells transfected with a plasmid encoding full length EGFP, green curves correspond to cells co-transfected with EGFP^{N}-CfaN and CfaC-EGFP^{C} (EGFP split at position 71), yellow curves correspond to cells co-transfected with EGFP^{N}-NpuN and NpuC-EGFP^{C} (EGFP split at position 71). Middle panel: Plot of Mean Fluorescence Intensity (MFI) obtained for each set of samples indicating the use of the Cfa intein allows to recover over 90% of the signal corresponding to full length EGFP. Right panel: plot representing the number of positive cells in each of the samples showing transfection efficency was comparable for all three sets.

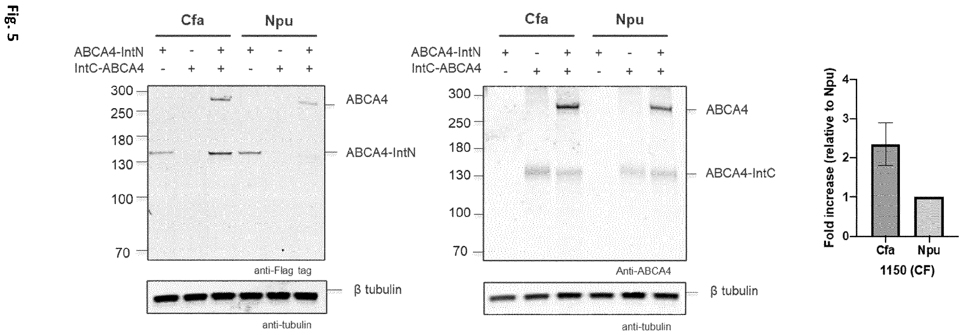

**Figure 5****:** Comparison of splicing yields of ABCA4 at position 1150 between Cfa and Npu. Cells were co-transfected with ABCA4^{N}1150-IntN and IntC-ABCA4^{C}1150, lysed and analyzed by WB. ABCA4^{N}1150 corresponds to the N-terminal fragment of ABCA4 ranging from residue 1 to 1149 and ABCA4^{C}1150 corresponds to the C-terminal fragment of ABCA4 ranging from residue 1150 to 2273. Constructs with either Cfa or Npu were tested. Left panel and middle panel show results blotted with an anti-FLAG tag and anti-ABCA4 mAb, respectively. Right panel corresponds to the densitometric quantification of the western blots.

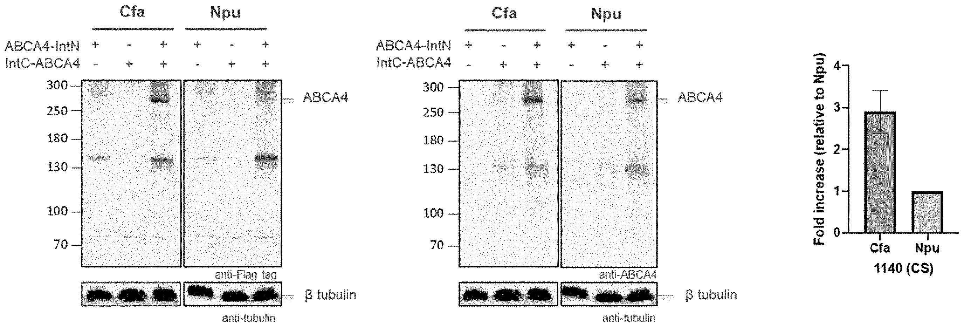

**Figure 6****.** Comparison of splicing yields of ABCA4 at position 1140 between Cfa and Npu. Cells were co-transfected with ABCA4^{N}1140-IntN and IntC-ABCA4^{C}1140, lysed and analyzed by WB. ABCA4^{N}1140 corresponds to the N-terminal fragment of ABCA4 ranging from residue 1 to 1139 and ABCA4^{C}1140 corresponds to the C-terminal fragment of ABCA4 ranging from residue 1140 to 2273. Constructs with either Cfa or Npu were tested. Left panel and middle panel show results blotted with an anti-FLAG tag and anti-ABCA4 mAb, respectively. Right panel corresponds to the densitometric quantification of the western blots.

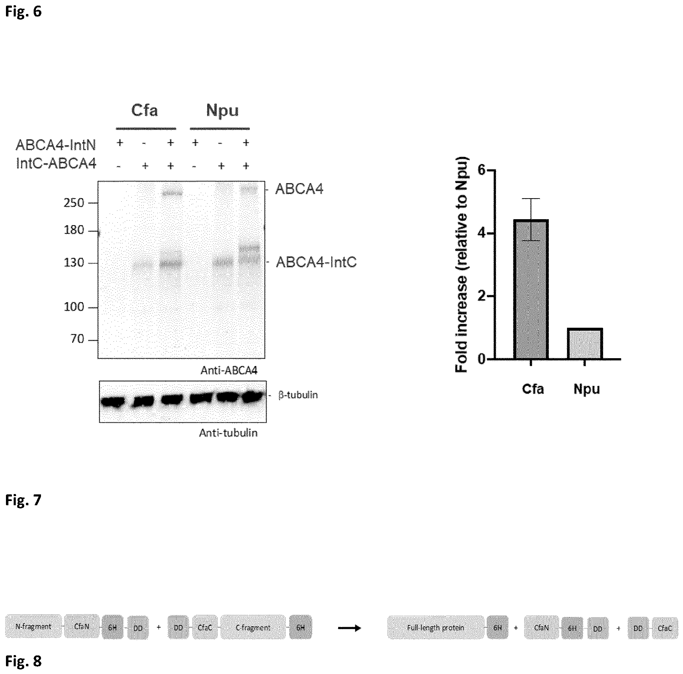

**Figure 7****:** Comparison of splicing yields of ABCA4 at position 1188 between Cfa and Npu. Cells were co-transfected with ABCA4^{N}1188-IntN and IntC-ABCA4^{C}1188, lysed and analyzed by WB. ABCA4^{N}1188 corresponds to the N-terminal fragment of ABCA4 ranging from residue 1 to 1187 and ABCA4^{C}1188 corresponds to the C-terminal fragment of ABCA4 ranging from residue 1188 to 2273. Constructs with either Cfa or Npu were tested. Left panel and middle panel show results blotted with an anti-FLAG tag and anti-ABCA4 mAb, respectively. Right panel corresponds to the densitometric quantification of the western blots.
**Figure 8****:** Reaction scheme of PTS including degrons. The N- terminal fragment of the protein of interest is fused to the IntN a linker, which in this case is a His6 tag, and a degron. The C-terminus of the protein is fused to the IntC, which in its N-terminus is linked to a degron. Splicing reaction results in the formation of the desired full-length protein, without any degron fused to it, and the cleaved inteins, each of them bearing a degradation signal.

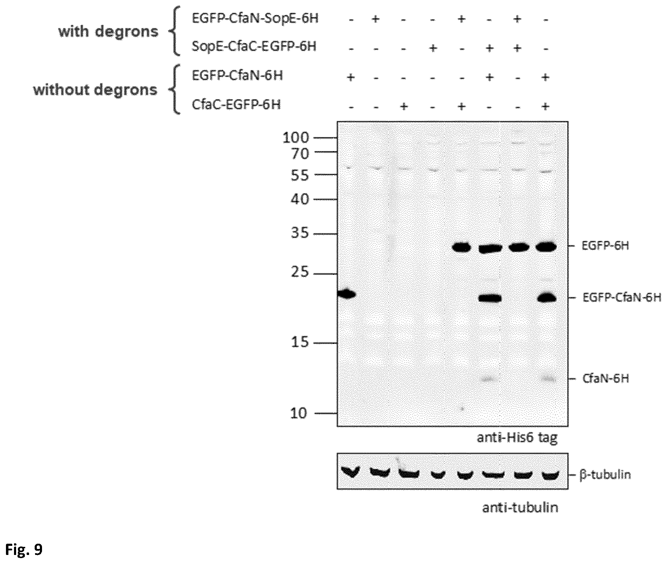

**Figure 9****:** Combination of inteins and degrons to reconstitute reporter protein EGFP. HEK293 cells were transfected with the indicated constructs, lysed and analyzed by western blot using an anti-His6 tag mAb. Results indicate that the use of constructs combining CfaN or C inteins with a degradation signal resulted in diseapearence of any signal arising from starting materials (EGFP-CfaN-6H) as well as from the intein (CfaN-6H).

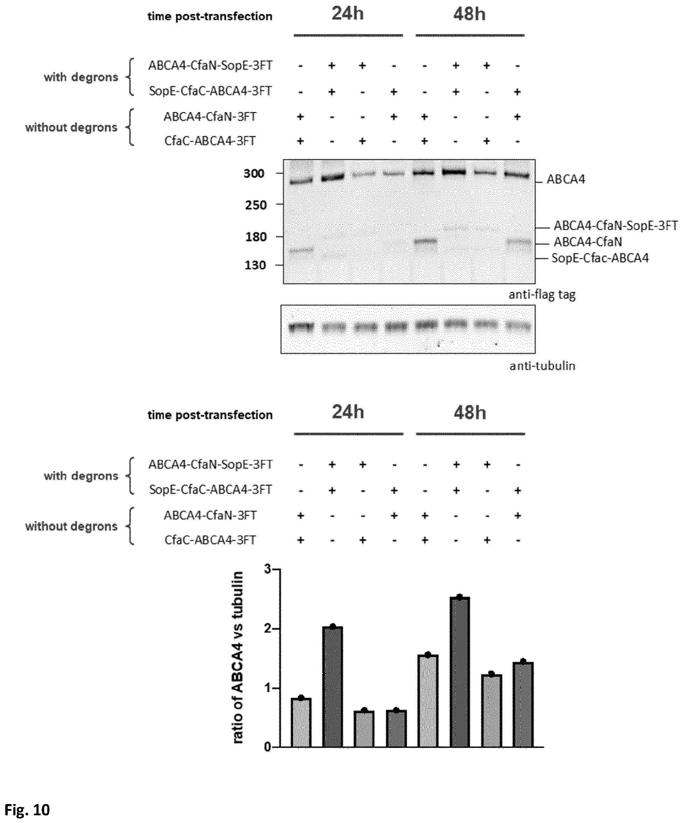

**Figure 10****:** Combination of inteins and degrons to reconstitute large protein ABCA4. HEK293 cells were transfected with ABCA4^{N}1150-CfaN and CfaC-ABCA4^{C}1150, with and without degrons, lysed and analyzed by western blot using an anti-FLAG tag mAb. Cells at two different timepoints were taken (24 and 48h). Top panel: western blot analysis. Bottom panel: densitometric quantification of western blot. Results indicate that the use of constructs combining Cfa N or C inteins with a degradation signal resulted in diseapearence of signal arising from starting materials (ABCA4-CfaN and CfaC-ABCA4). Interestingly, results also demonstrate that the combination of the Cfa intein with a degradation signal results in an increase in the yields of reconstituted full length ABCA4.

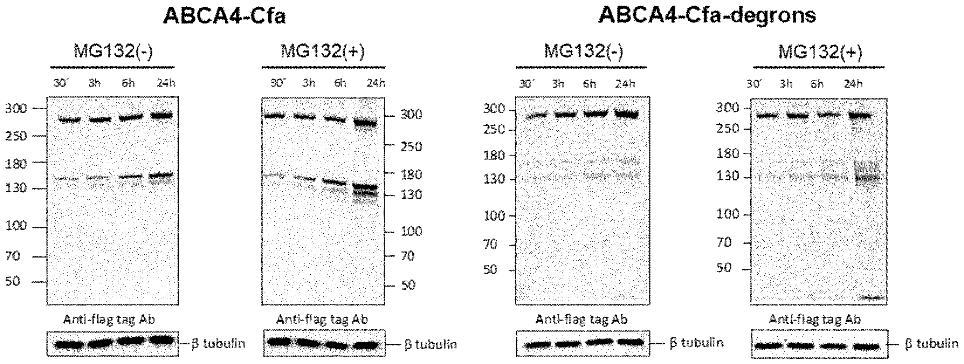

**Figure 11****:** Effect of intein-degron combination is mediated by the proteosome. Cells were co-transfected with ABCA4^{N}1150-CfaN and CfaC-ABCA4^{C}1150 constructs, with and without degrons; after 24h cells were incubated for the indicated amount of time (30 min, 3, 6 and 24h) in the presence, or absence, of the proteosome inhibitor MG132. After the indicated amount of time cells were lysed and analyzed by western blot using an anti-FLAG mAb.

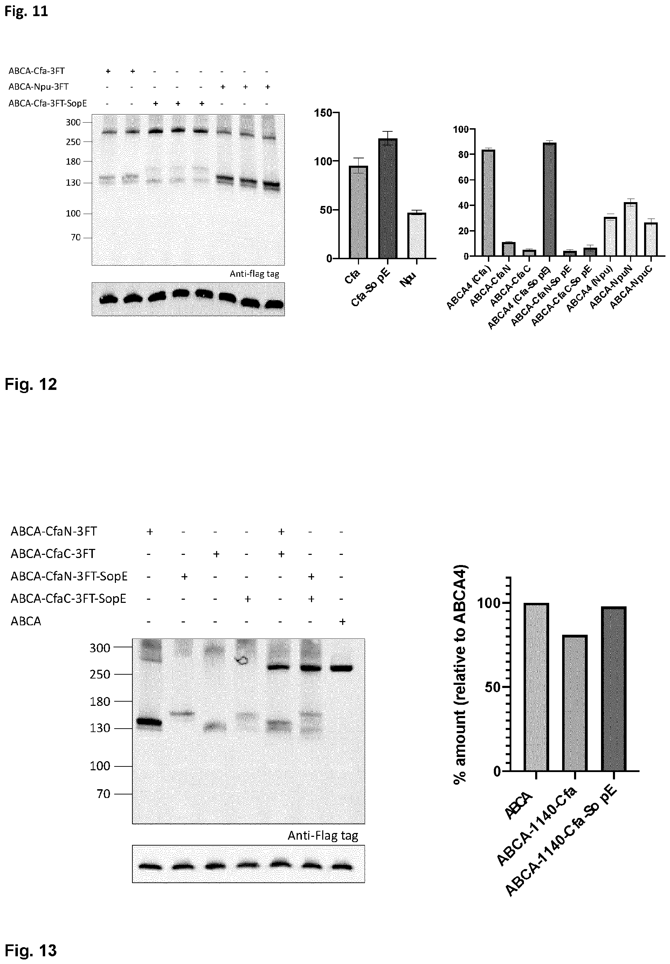

**Figure 12****:** Comparison of reconstitution yields using different inteins or inteins combined with degrons. Left panel: Western blot of reconstitution via PTS of ABCA4 split at position 1150 using Cfa, Cfa-SopE or Npu. Cells were co-transfected with ABCA4^{N}1150-CfaN and CfaC-ABCA4^{C}1150 (Cfa), ABCA4^{N}1150-CfaN-SopE and SopE-CfaC-ABCA4^{C}1150 (Cfa-SopE), or ABCA4^{N}1150-NpuN and NpuC-ABCA4^{C}1150 (Npu). After 48h cells were collected, lysed and analysed by WB. A duplicate for the Cfa constructs and a triplicate for the CfaSopE and Npu were analyzed. Right panel: Densitometry quantification of the product obtained with each combination of inteins and degrons, as well as remaining starting materials. Results show how reconstitution of product is higher when using Cfa combined with a degron (SopE). Interestingly, data shows that reconstitution with Npu is the lowest, but also that it is the one with the highest level of unreacted starting materials left over.
**Figure 13****:** Reconstitution of ABCA4 at position 1140 using CfaCmut, with and without degradation signal. Left panel: Cells were co-transfected with ABCA4^{N}1140-CfaN and CfaCmut-ABCA4^{C}1140, ABCA4^{N}1140-CfaN-SopE and SopE-CfaC-ABCA4^{C}1140 (Cfa-SopE), or full length ABCA4 (ABCA). After 48h cells were collected, lysed and analysed by WB using an anti-FLAG tag mAb. Right panel: densitometric quantification of level of ABCA4 reconstitution, compared to transfection with full length ABCA4.

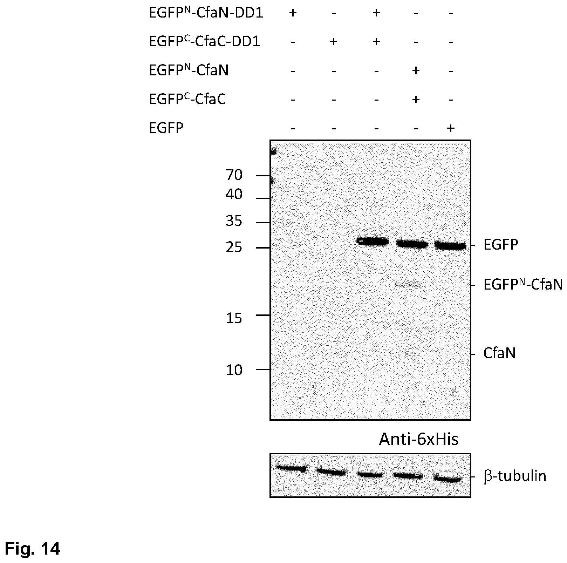

**Figure 14****:** Combination of inteins and degrons to reconstitute reporter protein EGFP, using DD1 degron. HEK293 cells were transfected with the indicated constructs, lysed and analyzed by western blot using an anti-His6 tag mAb. Combination if the Cfa inteins with DD1 degron results in elimination of undesired starting materials and excised intein.

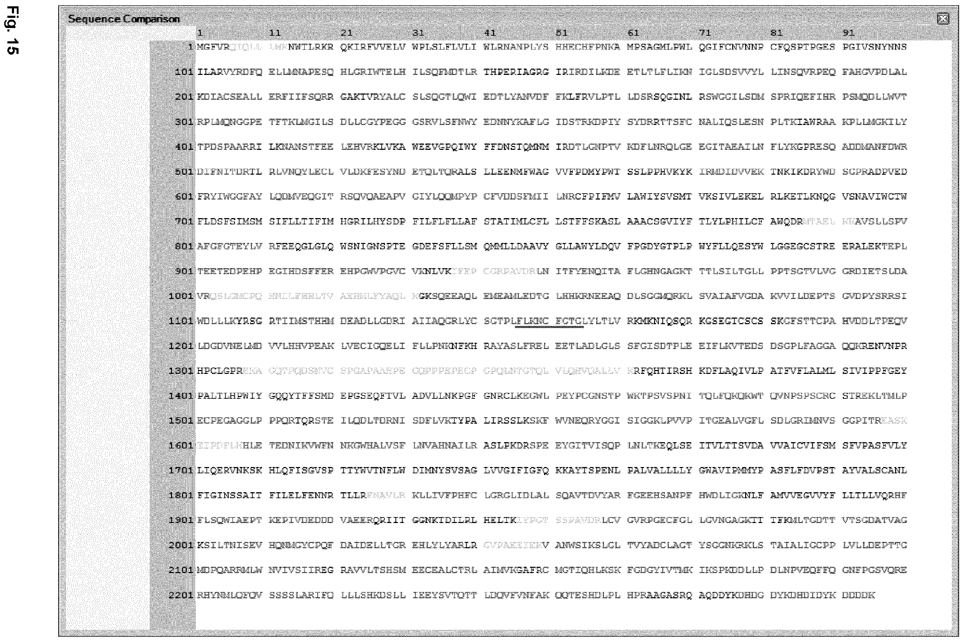

**Figure 15****:** Mass spectrometry analysis. Cells were transfected with ABCA4^{N}1150-CfaN-SopE and SopE-CfaC-ABCA4^{C}1150, and after 48h were collected and lysed. Cell lysate was run in an SDSPAGE gel and a band around 300KDa was cut, proteolyzed (see materials and methods) and analysed by LC-MS/MS. Results are summarized in the figure. In green are shown peptides identified by MSMS with a False Discovery Rate (FDR) below 1%. In yellow peptides identified with FDR below 5%. Underlined in red is shown the sequence of the split site.

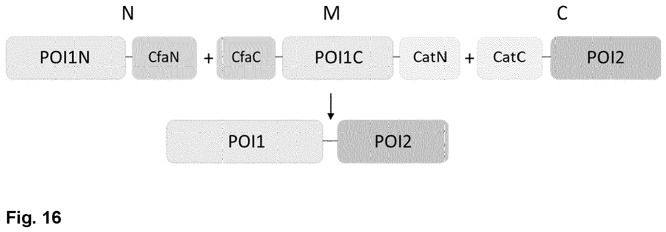

**Figure 16****:** Three-piece ligation using orthogonal consensus inteins.

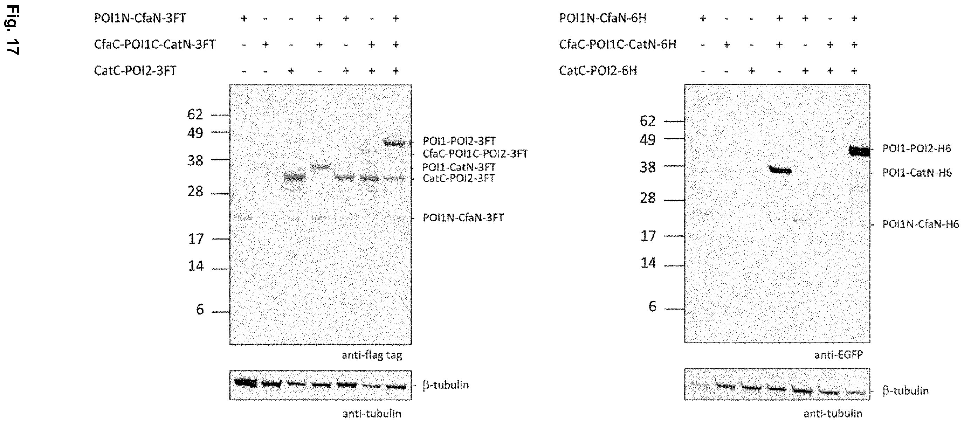

**Figure 17****:** Three-piece ligation using orthogonal consensus inteins. The N, M and C constructs shown in Figure 16 were transfected in HEK293 either alone, co-transfected in pairs, or the three fragments co-transfected together. Cells were lysed and analyzed by Western blot using an antibody against a 3FT tag present at the C-terminus of the N, M- and C-terminal fragments (left panel) or an antibody against the POIN-fragment (right).

### Description of the invention

The present invention relates to methods of use of engineered or naturally occurring split inteins, as well as their combination with degradation signals (destabilizing domains, degrons) to reconstitute large genes for gene therapy.

As used herein, the term "intein" means a naturally-occurring or artificially-constructed polypeptide sequence capable of catalyzing a protein splicing reaction that excises the intein sequence from a precursor protein and joins the flanking sequences (N- and C-exteins) with a peptide bond. They are typically 150-550 amino acids in size and may also contain a homing endonuclease domain. A list of known inteins is published at http://www.inteins.com and https://inteins.biocenter.helsinki.fi/index.php
The term "split intein" as used herein refers to any intein in which the N-terminal and C-terminal amino acid sequences are not directly linked via a peptide bond, such that the N-terminal and C-terminal sequences become separate fragments that can non-covalently re-associate, or reconstitute, into an intein that is functional for trans-splicing reactions.

The term "peptide bond" refers to a covalent chemical bond -CO-NH- formed between two molecules when the carboxy part of one molecule, referred to as a carboxy component, reacts with the amino part of another molecule, referred to as an amino component, causing the release of a molecule. For example, proteinogenic L-amino acids can form the peptide bond upon joining with the release of a molecule of water. Therefore, proteins and peptides can be regarded as chains of amino acid residues held together by peptide bonds. A peptide bond is an "amide bond" or "amide linkage".

The terms "polypeptide", "peptide" or "protein" are used interchangeably herein to refer to polymers of amino acids.

The term "amino acid" refers to naturally occurring and synthetic amino acids, as well as amino acid analogs and amino acid mimetics that function in a manner similar to the naturally occurring amino acids. Furthermore, the term "amino acid" includes both D- and L-amino acids (stereoisomers).

The term "natural amino acids" or "naturally occurring amino acid" comprises the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine.

As used herein the term "non-natural amino acid" or "synthetic amino acid" refers to a carboxylic acid, or a derivative thereof, substituted at position "a" with an amine group and being structurally related to a natural amino acid. Illustrative non- limiting examples of modified or uncommon amino acids include 2-aminoadipic acid, 3-aminoadipic acid, beta-alanine, 2-aminobutyric acid, 4-aminobutyric acid, 6-aminocaproic acid, 2-aminoheptanoic acid, 2-aminoisobutyric acid, 3-aminoisobutyric acid, 2-aminopimelic acid, 2,4-diaminobutyric acid, desmosine, 2,2'-diaminopimelic acid, 2,3-diaminopropionic acid, N-ethylglycine, N-ethylasparagine, hydroxy lysine, alio hydroxy lysine, 3-hydroxyproline, 4-hydroxyproline, isodesmosine, alloisoleucine, N-methylglycine, N-methyliso leucine, 6-N-methyl-lysine, N-methylvaline, norvaline, norleucine, ornithine, p-acetylphenylalanina, p-halophenylalanina, p-proparglyoxyphenylalanina, p-azidophenylalanina, p-benzoylphenylalanina, etc. This group also includes the D-isomers of the "natural amino acids".

As used herein, the term "split intein N-fragment" or "N-terminal split intein" or "N-terminal intein fragment" or "N-terminal intein sequence" (abbreviated "Int N")" refers to any intein sequence that comprises an N-terminal amino acid sequence that is functional for trans-splicing reactions, that is, that is capable of associating with a functional split intein C-fragment to form a complete intein that is capable of excising itself from the host protein, catalyzing the ligation of the extein or flanking sequences with a peptide bond, or that upon association with a split intein C-fragment catalyzes the "N-terminal cleavage", that is, the nucleophilic attack of the peptide bond between the extein and the N-terminus of the split intein N-fragment resulting in the breaking of said peptide bond. An IntN thus also comprises a sequence that is spliced out when trans-splicing occurs. An IntN can comprise a sequence that is a modification of the N-terminal portion of a naturally occurring intein sequence. For example, it can comprise additional amino acid residues and/or mutated residues so long as the inclusion of such additional and/or mutated residues does not render the IntN non-functional in trans-splicing. Preferably, the inclusion of the additional and/or mutated residues improves or enhances the trans-splicing activity of the IntN.

As used herein, the term "Degrons" means a naturally-occurring or artificially-constructed polypeptide sequence which when recombinantly fused to another polypeptide it accelerates its protein degradation via de proteosomal degradation pathway, or any other cellular degradation mechanism.

As interchangeably used herein, the terms "split intein C-fragment", "C-terminal split intein", "C-terminal intein fragment" and "C-terminal intein sequence" (abbreviated "lntC") refer to any intein sequence that comprises a C-terminal amino acid sequence that is functional for trans-splicing reactions, that is, that upon association is capable of associating with a functional split intein N-fragment to form a complete intein that is capable of excising itself from the host protein, catalyzing the ligation of the extein or flanking sequences with a peptide bond, or that upon association with a split N-intein catalyzes the "C-terminal cleavage", that is, the nucleophilic attack of the peptide bond between the extein and the C-terminus of the split intein C-fragment resulting in the breaking of said peptide bond. An IntC thus also comprises a sequence that is spliced out when trans-splicing occurs. An IntC can comprise a sequence that is a modification of the C-terminal portion of a naturally occurring intein sequence. For example, it can comprise additional amino acid residues and/or mutated residues so long as the inclusion of such additional and/or mutated residues does not render the IntC non-functional in trans-splicing. Preferably, the inclusion of the additional and/or mutated residues improves or enhances the trans-splicing activity of the IntC.

It is herein noted that, in the context of the present invention, "the N-terminal fragment of a protein to be reconstituted" refers to the N-terminal fragment of a protein, more particularly, a fragment of a protein of more than 25 KDa, more than 50 KDa or more than 100 KDa. The term "N-terminal fragment of a protein", as used herein, thus refers to a fragment of variable length that includes the N-terminus of the protein (in its mature or immature form). In a particular embodiment, the N-terminal fragment is a fragment comprising less than 100%, less than 90%, less than 80%, less than 70%, less than 60%, less than 50%, less than 40%, less than 30%, less than 20%, less than 10%, less than 5% of the length of the whole protein.

It is herein noted that, in the context of the present invention, "the C-terminal fragment of a protein to be reconstituted" refers to the C-terminal fragment of a protein, more particularly, a fragment of a protein of more than 25 KDa, more than 50 KDa or more than 100 KDa. The term "C-terminal fragment of a protein", as used herein, thus refers to a fragment of variable length that includes the C-terminus of the protein. In a particular embodiment, the C-terminal fragment is a fragment comprising less than 100%, less than 90%, less than 80%, less than 70%, less than 60%, less than 50%, less than 40%, less than 30%, less than 20%, less than 10%, less than 5% of the length of the whole protein.

As used herein, the term "polynucleotide" refers to a polymer composed of a multiplicity of nucleotide units (deoxyribonucleotides or ribonucleotides, or related structural variants or synthetic analogues thereof) linked via phosphodiester bonds (or related structural variants on synthetic analogues thereof). The term polynucleotide includes double or single stranded genomic and cDNA, RNA, any synthetic and genetically manipulated polynucleotide, and both sense and anti-sense polynucleotide (although only sense stands are being disclosed in the present invention). This includes single- and double-stranded molecules, i.e., DNA-DNA, DNA-RNA and RNA-RNA hybrids.

### Split intein N-fragments

Split intein N-fragment directly linked via a peptide bond to the N-terminal fragment of a protein to be reconstituted. The construct would have the general architecture from the N to the C-terminus:
(N-terminal fragment of the protein to be reconstituted) - InteinN.

The protein to be reconstituted can be any large gene, whose reconstitution could result in a positive therapeutic effect. A non-limited example of proteins that could be reconstituted with the instant invention, is the ABCA4 protein, and the proteins encoded by the genes listed in the following table 1. The goal of reconstituting these proteins is to treat diseases associated with mutations in their encoding genes, by providing correct versions of such genes. Additionally, the protein to be reconstituted could be a protein or enzyme to treat a disease, but not necessarily to replace a mutated one.

**Table 1.**

| Disease | Protein (gene) | Uniprot ID | Coding region Size |
|---|---|---|---|
| Usher Syndrome 2C (USH2C) | Adhesion G-protein coupled receptor V1 (ADGRV1) | Q8WXG9 | 18.9 |
| Macular degeneration, age-Ireated, 1 (ARMD1) | Hemicentin-1 (HMCN1) | Q96RW7 | 18.9 |
| Febrile seizures, familial, 4 (FEB4) | Adhesion G-protein coupled receptor V1 (ADGRV1) | Q8WXG9 | 18.9 |
| Usher Syndrome 2A (USH2A) | Usherin (USH2A) | 075445 | 15.6 |
| Retinitis pigmentosa 39 (RP39) | Usherin (USH2A) | 075445 | 15.6 |
| Alstom Syndrome | Alstrom syndrome protein 1 (ALMS1) | Q8TCU4 | 12.5 |
| Duchenne Muscular dystrophy | Dystrophin (DMD) | P11532 | 11.6 |
| Deafness, autosomal recessive, 3 (DFNB3) | Unconventional myosin-XV (MYO15A) | Q9UKN7 | 10.8 |
| Usher syndrome 1D/F (USH1DF) | Cadherin-23 (CDH23) | Q9H251 | 10.1 |
| Usher syndrome 1D (USH1D) | Cadherin-23 (CDH23) | Q9H251 | 10.1 |
| Pituitary adenoma 5, multiple types (PITA5) | Cadherin-23 (CDH23) | Q9H251 | 10.1 |
| Deafness, autosomal recessive, 12 (DFNB12) | Cadherin-23 (CDH23) | Q9H251 | 10.1 |
| Retinitis pigmentosa 25 (RP25) | Protein eyes shit homolog (EYS) | Q5T1H1 | 9.4 |
| Muscular dystrophy, limb-girdle ar 23 (LGMDR23) | Laminin subunit alpha-2 (LAMA2) | P24043 | 9.4 |
| Merosin-deficient congenital muscular dystrophy 1A (MDC1A) | Laminin subunit alpha-2 (LAMA2) | P24043 | 9.4 |
| Von Willebrand disease 1 (VWD1) | von Willebrand factor (VWF) | P04275 | 8.4 |
| Von Willebrand disease 2 (VWD2) | von Willebrand factor (VWF) | P04275 | 8.4 |
| Von Willebrand disease 3 (VWD3) | von Willebrand factor (VWF) | P04275 | 8.4 |
| Transient bullous dermolysis of the newborn (TBDN) | Collagen alpha-1(VII) chain (COL7A1) | Q02388 | 8.1 |
| Nail disorder, no-syndromic congenital, 8 (NDNC8) | Collagen alpha-1 (VII) chain (COL7 A1) | Q02388 | 8.1 |
| Epidermolysis bullosa dystrophica, with subcorneal cleavage (EBDSC) | Collagen alpha-1 (VII) chain (COL7 A1) | Q02388 | 8.1 |
| Epidermolysis bullosa dystrophica, Bart type (B-DEB) | Collagen alpha-1 (VII) chain (COL7A1) | Q02388 | 8.1 |
| Epidemolysis bullosa pruriginosa (EBP) | Collagen alpha-1 (VII) chain (COL7A1) | Q02388 | 8.1 |
| Epidemolysis bullosa dystrophica, pretibial type (PR-DEB) | Collagen alpha-1 (VII) chain (COL7A1) | Q02388 | 8.1 |
| Epidemolysis bullosa dystrophica ar (RDEB) | Collagen alpha-1 (VII) chain (COL7 A1) | Q02388 | 8.1 |
| Epidemolysis bullosa dystrophica ad (DDEB) | Collagen alpha-1 (VII) chain (COL7A1) | Q02388 | 8.1 |
| Spinocerebellar ataxia 6 (SCA6) | Voltage-dependent P/Q-type calcium channel subunitalpha-1A (CACNA1A) | O00555 | 7.7 |
| Migraine, familial hemiplegic, 1 (FHM1) | Voltage-dependent P/Q-type calcium channel subunit alpha-1A (CACNA1A) | 000555 | 7.7 |
| Episodic ataxia 2 (EA2) | Voltage-dependent P/Q-type calcium channel subunit alpha-1A (CACNA1A) | 000555 | 7.7 |
| Epileptic encephalopathy, early infantile, 42 (EIEE42) | Voltage-dependent P/Q-type calcium channel subunit alpha-1A (CACNA1A) | 000555 | 7.7 |
| Senior-Loken syndrome 6 (SLSN6) | Centrosomal protein of 290 kDa (CEP290) | 015078 | 7.5 |
| Meckel syndrome 4 (MKS4) | Centrosomal protein of 290 kDa (CEP290) | 015078 | 7.5 |
| Leber Congenital Amaurosis 10 (LCA10) | Centrosomal protein of 290 kDa (CEP290) | 015078 | 7.5 |
| Joubert syndrome 5 (JBTS5) | Centrosomal protein of 290 kDa (CEP290) | 015078 | 7.5 |
| Bardet-Biedl syndrome 14 (BBS14) | Centrosomal protein of 290 kDa (CEP290) | 015078 | 7.5 |
| Occult macular dystrophy (OCMD) | Retinitis pigmentosa 1-like 1 protein (RP1L1) | Q8IWN7 | 7.2 |
| Hyperaldosteronism, familial, 4 (HALD4) | Voltage-dependent T-type calcium channel subunit alpha-1H (CACNA1H) | 095180 | 7.1 |
| Hemophilia A | Coagulation factor FVIII | P00451 | 7.1 |
| Epilepsy, idiopathic generalized 6 (EIG6) | Voltage-dependent T-type calcium channel subunit alpha-1H (CACNA1H) | 095180 | 7.1 |
| Epilepsy, childhood absence 6 (ECA6) | Voltage-dependent T-type calcium channel subunit alpha-1H (CACNA1H) | 095180 | 7.1 |
| Deafness, ar, 28 (DFNB28) | TRIO and F-actin-binding protein (TRIOBP) | Q9H2D6 | 7.1 |
| Retinitis pigmentosa 13 (RP13) | Pre-mRNA-processing-splicing factor 8 (PRPF8) | Q6P2Q9 | 7.0 |
| Stargardt disease 1 (STGD1) | Retinal-specific phospholipid-transporting ATPase ABCA4 (ABCA4) | P78363 | 6.8 |
| Retinitis pigmentosa 19 (RP19=) | Retinal-specific phospholipid-transporting ATPase ABCA4 (ABCA4) | P78363 | 6.8 |
| Macular degeneration, age-related, 2 (ARMD2) | Retinal-specific phospholipid-transporting ATPase ABCA4 (ABCA4) | P78363 | 6.8 |
| Fundus flavimaculatus (FFM) | Retinal-specific phospholipid-transporting ATPase ABCA4 (ABCA4) | P78363 | 6.8 |
| Cone-rod dystrophy 3 (CORD3) | Retinal-specific phospholipid-transporting ATPase ABCA4 (ABCA4) | P78363 | 6.8 |
| Usher syndrome 1B (USH1B) | Unconventional myosin-VIIa (MYO7A) | Q13402 | 6.7 |
| Deafness ar 2 (DFNB2) | Unconventional myosin-VIIa (MYO7A) | Q13402 | 6.7 |
| Deafness ad 11 (DFNA11) | Unconventional myosin-VIIa (MYO7 A) | Q13402 | 6.7 |
| Retinitis pigmentosa 1 (RP1) | Oxygen-regulated protein 1 (RP1) | P56715 | 6.5 |
| Muscular dystrophy, limb-girdle, ar 2 (LGMDR2) | Dysferlin (DYSF) | 075923 | 6.5 |
| Miyoshi muscular dystrophy 1 (MMD1) | Dysferlin (DYSF) | 075923 | 6.5 |
| Distal myopathy with anterior tibial onset (DMAT) | Dysferlin (DYSF) | 075923 | 6.5 |
| Deafness, ad, 12 (DFNA12) | Alpha-tectorin (TECTA) | 075443 | 6.5 |
| Deafness, ar, 21 (DFNB21) | Alpha-tectorin (TECTA) | 075443 | 6.5 |
| Retinitis pigmentosa 33 (RP33) | U5 small nuclear ribonucleoprotein 200 kDa helicase (SNRNP200) | 075643 | 6.4 |
| Progressive familial heart block 1A (PFHB1A) | Sodium channel protein type 5 subunit alpha (SCN5A) | Q14524 | 6.2 |
| Long QT syndrome 3 (LQT3) | Sodium channel protein type 5 subunit alpha (SCN5A) | Q14524 | 6.2 |
| Brugada syndrome 1 (BRGDA1) | Sodium channel protein type 5 subunit alpha (SCN5A) | Q14524 | 6.2 |
| Sick sinus syndrome 1 (SSS1) | Sodium channel protein type 5 subunit alpha (SCN5A) | Q14524 | 6.2 |
| Familial paroxysmal ventricular fibrillation 1 (VF1) | Sodium channel protein type 5 subunit alpha (SCN5A) | Q14524 | 6.2 |
| Sudden infant death syndrome (SIDS) | Sodium channel protein type 5 subunit alpha (SCN5A) | Q14524 | 6.2 |
| Atrial standstill 1 (ATRST1) | Sodium channel protein type 5 subunit alpha (SCN5A) | Q14524 | 6.2 |
| Cardiomyopathy, dilated 1E (CMD1E) | Sodium channel protein type 5 subunit alpha (SCN5A) | Q14524 | 6.2 |
| Atrial fibrillation, familial, 10 (ATFB10) | Sodium channel protein type 5 subunit alpha (SCN5A) | Q14524 | 6.2 |
| Seizures, benign familial infantile, 3 (BFIS3) | Sodium channel protein type 2 subunit alpha (SCN2A) | Q99250 | 6.2 |
| Migraine, familial hemiplegic, 3 (FHM3) | Sodium channel protein type 1 subunit alpha (SCN1A) | P35498 | 6.2 |
| Intractable childhood epilepsy with generalized tonic-clonic seizures (ICEGTC) | Sodium channel protein type 1 subunit alpha (SCN1A) | P35498 | 6.2 |
| Infantile spasms | Sodium channel protein type 2 subunit alpha (SCN2A) | Q99250 | 6.2 |
| Generalized epilepsy with febrile seizures plus 2 (GEFS+2) | Sodium channel protein type 1 subunit alpha (SCN1A) | P35498 | 6.2 |
| Febrile seizures, familial, 3A (FEB3A) | Sodium channel protein type 1 subunit alpha (SCN1A) | P35498 | 6.2 |
| Epileptic enfephalopathy, early infantile, 6 (EIEE6) | Sodium channel protein type 1 subunit alpha (SCN1A) | P35498 | 6.2 |
| Epileptic encephalopathy, early infantile, 62 (EIEE62) | Sodium channel protein type 3 subunit alpha (SCN3A) | Q9NY46 | 6.2 |
| Epileptic encephalopathy, early infantile, 11 (EIEE11) | Sodium channel protein type 2 subunit alpha (SCN2A) | Q99250 | 6.2 |
| Epilepsy, familial focal, with variable foci 4 (FFEVF 4) | Sodium channel protein type 3 subunit alpha (SCN3A) | Q9NY46 | 6.2 |
| Dravet Syndrome | Sodium channel protein type 1 subunit alpha (SCN1A) | P35498 | 6.2 |
| Deafness, ar, 9 (DFNB9) | Otoferlin (OTOF) | Q9HC10 | 6.2 |
| Auditory neuropathy, ar, 1 (AUNB1) | Otoferlin (OTOF) | Q9HC10 | 6.2 |
| Deafness, ad, 4A (DFNA4A) | Myosin-14 (MYH14) | Q7Z406 | 6.2 |
| Peripheral neuropathy, myopathy, hoarseness and hearing liss (PNMHH) | Myosin-14 (MYH14) | Q7Z406 | 6.2 |
| Seizures, benign familial infantile, 5 (BFIS5) | Sodium channel protein type 8 subunit alpha (SCN8A) | Q9UQD0 | 6.1 |
| Myoclonus, familial, 2 (MYOCL2) | Sodium channel protein type 8 subunit alpha (SCN8A) | Q9UQD0 | 6.1 |
| Epileptic encephalopathy, early infantile, 13 (EIEE13) | Sodium channel protein type 8 subunit alpha (SCN8A) | Q9UQD0 | 6.1 |
| Cognitive impairment with or without cerebellar ataxia (CIAT) | Sodium channel protein type 8 subunit alpha (SCN8A) | Q9UQD0 | 6.1 |
| Night blindness, congenital stationary 2A (CSNB2A) | Voltage-dependent L-type calcium channel subunit alpha-1F (CACNA1F) | 060840 | 6.0 |
| Cone-rod dystrophy, X-linked 3 (CORDX3) | Voltage-dependent L-type calcium channel subunit alpha-1F (CACNA1F) | 060840 | 6.0 |
| Aaland island eye disease (AIED) | Voltage-dependent L-type calcium channel subunit alpha-1F (CACNA1F) | 060840 | 6.0 |
| Usher syndrome 1F (USH1F) | Photocadherin-15 (PCDH15) | Q96QU1 | 5.9 |
| Usher syndrome 1D/F (USH1DF) | Photocadherin-15 (PCDH15) | Q96QU1 | 5.9 |
| Deafness, ar, 23 (DFNB23) | Photocadherin-15 (PCDH15) | Q96QU1 | 5.9 |
| Deafness, ar, 16 (DFNB16) | Stereocilin (STRC) | Q7RTU9 | 5.2 |
| Deafness-infertility syndrome (DIS) | Stereocilin (STRC) | Q7RTU9 | 5.2 |
| Otospondylomegaepiphys eal dysplasia ad (OSMEDA) | Collagen alpha-2(XI) chain (COL11A2) | P13942 | 4.7 |
| Otospondylomegaepiphys eal dysplasia ar (OSMEDB) | chain (COL11A2) | P13942 | 4.7 |
| Deafness ad 13 (DFNA13) | Collagen alpha-2(XI) chain (COL11A2) | P13942 | 4.7 |
| Deafness ar 53 (DFNB53) | Collagen alpha-2(XI) chain (COL11A2) | P13942 | 4.7 |
| Fibrochondrogenesis 2 (FBCG2) | Collagen alpha-2(XI) chain (COL11A2) | P13942 | 4.7 |
| Cystic Fibrosis (CF) | Cystic fibrosis transmembrane conductance regulator (CFTR) | P13569 | 4.6 |
| Congenital bilateral absence of the vas deferens (CBAVD) | Cystic fibrosis transmembrane conductance regulator (CFTR) | P13569 | 4.6 |
| Fanconi anemia, complementation group A (FANCA) | Fanconi anemia group A protein (FANCA) | 015360 | 4.4 |
| Retinitis pigmentosa 12 (RP12) | Protein crumbs homolog 1 (CRB1) | P82279 | 4.2 |
| Pigmented paravenous chorioretinal atrophy (PPCRA) | Protein crumbs homolog 1 (CRB1) | P82279 | 4.2 |
| Leber congenital amaurosis 8 (LCA8) | Protein crumbs homolog 1 (CRB1) | P82279 | 4.2 |
| Deafness ad 22 (DFNA22) | Unconventional myosin-VI (MYO6) | Q9UM54 | 4.1 |
| Deafness ar 37 (DFNB37) | Unconventional myosin-VI (MYO6) | Q9UM54 | 4.1 |
| Deafness ad 22, with hypertrophic cardiomyopathy (DFNHCM) | Unconventional myosin-VI (MYO6) | Q9UM54 | 4.1 |
| Leber congenital amaurosis 6 (LCA6) | X-linked retinitis pigmentosa GTPase regulator-interacting protein 1 (RPGRIP1) | Q96KN7 | 4.0 |
| Cone-rod dystrophy 13 (CORD13) | X-linked retinitis pigmentosa GTPase regulator-interacting protein 1 (RPGRIP1) | Q96KN7 | 4.0 |

| | | | |
|---|---|---|---|
| LCA = leber congenital amaurosis RP = retinitis pigmentosa ad = autosomal dominant ar = autosomal recessive PR = photoreceptors RPE = retinal pigment epithelium ECM = extracellular matrix | | | |

Therefore, a first aspect the invention refers to a Split intein N-fragment directly linked via a peptide bond to the N-terminal fragment of a protein to be reconstituted, wherein, as reflected above, the IntN is linked, either directly or by using a linker, to the C-terminus of the N terminal fragment.
(from hereinafter **"the split intein N-fragment of the invention"**)**.**

A preferred embodiment of the first aspect of the invention refers to a split intein N-fragment directly linked via a peptide bond to a degron (from hereinafter **"the split intein N-fragment degron of the invention"**)**,** wherein the degron is linked to the intein N-fragment via the C-terminus of the intein, with or without a linker between the intein N-fragment and the degron, and wherein the N-terminus of the Split intein N-fragment is directly linked via a peptide bond to the N-terminal fragment of a protein to be reconstituted.

Therefore, the architecture of the construct of the split intein N-fragment degron of the invention, from the N to the C-terminus would be:
(N-terminal portion of the protein to be reconstituted)-(Intein-N fragment) - (Degron).

If a linker is introduced between the IntN and the degron then the architecture of the construct of the split intein N-fragment degron of the invention, from the N to the C-terminus would be:
(N-terminal portion of the protein to be reconstituted)-(Intein-N fragment) - (linker) - (Degron)

Preferably, the Intein N of any **of the split intein N-fragment of the invention** or **the split intein N-fragment degron of the invention** is selected from any of the following listed in table 2 below:

**Table 2**

| | | |
|---|---|---|
| **Cfa^{N}** | CLSYDTEILTVEYGFLPIGKIVEERIECTVYTVDKNG FVYTQPIAQWHNRGEQEVFEYCLEDGSIIRATKDHKF MTTDGQMLPIDEIFERGLDLKQVDGLP | 27 |
| **Cat^{N}** | CLSGDTMIEILDDDGIIQKISMEDLYQRLA | 30 |
| **Npu^{N}** | CLSYETEILTVEYGLLPIGKIVEKRIECTVYSVDNNG NIYTQPVAQWHDRGEQEVFEYCLEDGSLIRATKDHKF MTVDGQMLPIDEIFERELDLMRVDNLPN | 32 |

wherein preferably the Intein N is the CfaN intein of SEQ ID NO 27 or any of its variants, or the Cat intein (SEQ ID NO 30), or any Cat, or Cfa variant with increased promiscuity, wherein promiscuity is understood as the ability of the intein to perform the protein trans-splicing reaction independently of the identity of the residues immediately adjacent to the split site.

Inteins promiscuity refers to their ability to perform the PTS reaction independently of the identity of the amino acids immediately adjacent to the split site, that is, the site where the intein is inserted in the protein of interest. Below is shown the schematic representation of a split site:

| | |
|---|---|
| -3 -2 -1 | +1+2+3 |
| X - X - X - Intein N | Intein C - X - X - X |

Typically inteins have strong preferences for certain amino acids in those positions. For example the Cat intein (SEQ ID NO 30) favours a Cys at position +1, and a Glu at position -1. More promiscous variants would be those able to perform the protein trans-splicing reaction in good yields even in the absence of such preferred residues.

In a particular embodiment, the split intein N-fragment comprises or consists of a variant of the amino acid sequence of SEQ ID NO: 27 having at least 90% sequence identity with SEQ ID NO: 27 over the whole sequence.

In a particular embodiment, the split intein N-fragment comprises or consists of a variant of the amino acid sequence of SEQ ID NO: 30 having at least 90% sequence identity with SEQ ID NO: 30 over the whole sequence.

The term "variant" as used herein refers to a polypeptide molecule that is substantially similar to a particular polypeptide sequence. The variant may be similar in structure and biological activity to the polypeptide from which it derives. Thus, the variant may refer to a mutant of a polypeptide sequence. The term "mutant" refers to a polypeptide molecule the sequence of which has one or more amino acids added, deleted, substituted or otherwise chemically modified in comparison to the polypeptide molecule from which it derives. The mutant may retain substantially the same properties as the polypeptide molecule from which it derives or lack the biological activity of the claimed sequences.

In a particular embodiment, intein variants include mutants in which the catalytic and second shell accelerator residues of the intein are maintained, and only non-catalytic residues, or residues outside the second shell are mutated. Second shell accelerator residues are those adjacent to the active site of the intein, which play a critical role in tuning the splicing activity of the inteins (Stevens, A. J., Brown, Z. Z., Shah, N. H., Sekar, G., Cowburn, D., & Muir, T. W. (2016). Journal of the American Chemical Society. http://doi.org/10.1021/jacs.5b13528).

The variant of the split intein N-fragment of SEQ ID NO: 27 has at least 90% sequence identity with SEQ ID NO: 27. In a particular embodiment, the variant of the split intein N-fragment of SEQ ID NO: 27 has at least 91%, at least 92%, at least 93%, at least 94%, at least 95%, at least 96%, at least 97%, at least 98%, at least 99% sequence identity with SEQ ID NO: 27.

The variant of the split intein N-fragment of SEQ ID NO: 30 has at least 90% sequence identity with SEQ ID NO: 30. In a particular embodiment, the variant of the split intein N-fragment of SEQ ID NO: 30 has at least 91%, at least 92%, at least 93%, at least 94%, at least 95%, at least 96%, at least 97%, at least 98%, at least 99% sequence identity with SEQ ID NO: 30.

The terms "identity", "identical", "percent identity" or "sequence identity" in the context of two or more amino acid or nucleotide sequences, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid sequences. One such non-limiting example of a sequence alignment algorithm is the algorithm described in Karlin et al., 1990, Proc. Natl. Acad. Sci., 87:2264-8, as modified in Karlin et al., 1993, Proc. Natl. Acad. Sci., 90:5873-7, and incorporated into the N BLAST and XBLAST programs (Altschul et al., 1991, Nucleic Acids Res., 25:3389-402). In certain embodiments, Gapped BLAST can be used as described in Altschul et al., 1997, Nucleic Acids Res. 25:3389-402. BLAST-2, WU-BLAST-2 (Altschul et al., 1996, Methods in Enzymology, 266:460-80), ALIGN, ALIGN-2 (Genentech, South San Francisco, California) or Megalign (DNASTAR) are additional publicly available software programs that can be used to align sequences. In certain alternative embodiments, the GAP program in the GCG software package, which incorporates the algorithm of Needleman and Wunsch (J. Mol. Biol. 48:444-53 (1970)) can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between amino acid sequences is determined using the algorithm of Myers and Miller (CABIOS, 4:1 1 -7 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second amino acid sequence is calculated as 100 x (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the global alignment taken the entirety of both sequences into consideration is used, therefore all letters and null in each sequence must be aligned. In this case, the same formula as above can be used but using as Z value the length of the region wherein the first and second sequence overlaps, said region having a length which is substantially the same as the length of the first sequence.

As a non-limiting example, whether any particular polypeptide has a certain percentage sequence identity (e.g., is at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, Wl 5371 1). Bestfit uses the local homology algorithm of Smith and Waterman, Advances in Applied Mathematics 2:482-9 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of nucleotides in the reference sequence are allowed.

The term "linker" between the split intein N-fragment and the degron refers to a sequence of amino acids that connect the C-terminus of the intein N-fragment and the N-terminus of the degron sequence. The linker would preferably be a polypeptide of 1 to 100 amino acids, or 1 to 5, or 1 to 10, or 1 to 50, or 1 to 25 amino acids. The linker could be a Gly rich peptide, or a Gly-Ser rich peptide. An example of a Gly-Ser rich peptide would be the sequence GGS, or polymers of this linker with the general formula (GGS)n, where n can go from 1 to 10. A general formula for the linker would be ((G)nS)y, where n goes from 1 to 5 and y from 1 to 10. Additionally, epitope tags can be used as linkers, for example the hexahistidine (His6, sequence: GHHHHHHG (SEQ ID NO 38) tag, or a triple flag tag (3FT, sequence: DYKDHDGDYKDHDIDYKDDDDK (SEQ ID NO 39)).

In another particular embodiment, the degron directly linked via a peptide bond to the split intein N-fragment is selected from any of those listed in table 3 below:

**Table 3**

| **Degron** | **Sequence** | SEQ ID NO |
|---|---|---|
| CL1 | ACKNWFSSLSHFVIHL | 40 |
| Deg1 | MNKIPIKDLLNPQITDEFKSSILDINKKLFSICCNLP KLPESVTTEEEVELRDILGFLSRAN | 41 |
| PEST | RSSSPSDSDTSGFSSGSDHLSDLISSLRIS | 42 |
| DD1 | RISFGSPPPMAXG; X=any amino acid | 43 |
| DD2 | GSPPPMAXG; X=any amino acid | 44 |
| DD3 | TNGILKLGCQG | 45 |
| M1 | CSEIIPMSRSTPISTMG | 46 |
| M2 | VSFAFNLNSLIVGILRFHW | 47 |
| SopE | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSA | 48 |
| SopE-1-78 | MTKITLSPQNFRIQKQETTLLKEKSTEKNSLAKSILA VKNHFIELRSKLSERFISHKNTESSATHFHRGSASEG RAVL | 49 |
| SopE-15-78 | QETTLLKEKSTEKNSLAKSILAVKNHFIELRSKLSER FISHKNTESSATHFHRGSASEGRAVL | 50 |
| SopE-15-50 | QETTLLKEKSTEKNSLAKSILAVKNHFIELRSKLS | 51 |
| L2 | SLISLPLPTRVKFSSLLLIRIMKIITMTFPKKLRS | 52 |
| L6 | FYYPIWFARVLLVHYQ | 53 |
| L9 | SNPFSSLFGASLLIDSVSLKSNWDTSSSSCLISFFSS VMFSSTTRS | 54 |
| L10 | CRQRFSCHLTASYPQSTVTPFLAFLRRDFFFLRHNSS AD | 55 |
| L11 | GAPHVVLFDFELRITNPLSHIQSVSLQITLIFCSLPS LILSKFLQV | 56 |
| L12 | NTPLFSKSFSTTCGVAKKTLLLAQISSLFFLLLSSNI AV | 57 |
| L15 | PTVKNSPKIFCLSSSPYLAFNLEYLSLRIFSTLSKCS NTLLTSLS | 58 |
| L16 | SNQLKRLWLWLLEVRSFDRTLRRPWIHLPS | 59 |
| M3 | KSVTLESRSPKFLNWFSVFSLFKVITTG | 34 |
| M4 | YMSILRCASGKISIAAPPYIF | 35 |
| M5 | AGESFNFMVKLLYKHPILPCLKTLLSIRSSCSPR | 37 |

Other non-limiting examples of degrons include polypeptides related to DHFR (dehydrofolate reductase), FKBP (FK506 Binding Protein), FRB (FKBP-Rapamycin binding protein) and PDE5 (phsophodiesterase type 5).

In yet another preferred embodiment, preferred combinations of split intein N-fragments and degrons are selected from the list consisting of: the CfaN intein and any of DD1, SopE, SopE1-78, SopE15-78, or SopE15-50. In yet another preferred embodiment, preferred combinations of split intein N-fragments and degrons are selected from the list consisting of the CfaN intein and SEQ ID NO 42, 43, 49, 50, 51, 54 or 34.

### Complex comprising a split intein N-fragment

In another aspect, the invention relates to any of the following complex/es: the split intein N-fragment of the invention or the split intein N-fragment degron of the invention, hereinafter first complex/es of the invention, wherein each of these complexes comprises:
(i) the N-terminal fragment of a protein of interest to be reconstituted, and
(ii) a split intein N-fragment (the split intein N-fragment of the invention) or a split intein N-fragment directly linked via a peptide bond to a degron, optionally through a linker, as defined in the above section (the split intein N-fragment degron of the invention);
wherein the complex/es optionally comprise a linker between (i) and (ii) and
wherein
- the N-terminal fragment of the protein of interest is linked to the N-terminus of the split intein N-fragment by an amide linkage, or
- if the complex/es comprise a linker, the N-terminal fragment of the protein of interest is bound to the linker by an amide linkage and/or the linker is bound to the N-terminus of the split intein N-fragment by an amide linkage.

Illustrative non-limitative examples of proteins of interest, useful in the any of the above complexes, are those shown in table 1 above. Further proteins of interest can be selected from antibodies, antibody fragments, including Fc domain, scFv, nanobodies, bi-specific antibodies, proteins, and, preferably, any proteins larger than 25, 50, 100 KDa.

As already indicated, optionally, the proteins of interest and the split intein N-fragment may be joined through a linker, so the linker is located in between the protein of interest and the N-intein. The nature of the linker will depend on the nature of the protein of interest. In a particular embodiment, the linker is a peptide. In a particular embodiment, the linker is a peptide having a length of 1, 2, 3, 4, 5, 10, 20, 50, 100 or more amino acid residues; specifically, it may be 1 to 3 amino acid residues. Preferably, the N-terminus of the linker is linked to the C-terminus of the protein of interest and the C-terminus of the linker is linked to the N-terminus of the N-intein through peptide bonds.

In another particular embodiment, the complex does not comprise a linker between the N-terminal fragment of a protein of interest and the split intein N-fragment. In this particular embodiment, the protein of interest is linked to the N-terminus of the split intein N-fragment by an amide linkage.

In another particular embodiment, if the complexes comprise a linker, the complex is a fusion protein. The term "fusion protein" is well known in the art, referring to a single polypeptide chain artificially designed which comprises two or more sequences from different origins, natural and/or artificial. The fusion protein, per definition, is never found in nature as such.

### Split intein C-fragment

In another aspect, the invention relates refers to a Split intein C-fragment directly linked via a peptide bond to the C-terminal fragment of a protein to be reconstituted (from hereinafter **"the split intein C-fragment of the invention"**)**.**

More preferably, the invention refers to a split intein C-fragment directly linked via a peptide bond to a degron (from hereinafter **"the split intein C-fragment degron of the invention"**)**,** wherein the degron is linked to the intein C-fragment via the N-terminus of the intein, with or without a linker between the intein C-fragment and the degron, and wherein the C-terminus of the Split intein C-fragment is directly linked via a peptide bond to the N-terminus of the C-terminal fragment of a protein to be reconstituted.

Therefore, the architecture of the construct of the split intein C-fragment degron of the invention, from the N to the C-terminus would be:
(Degron)-(Intein-C fragment) - (C- terminal portion of the protein to be reconstituted)

If a linker is included between Degron and Intein C, the architecture of the construct of the split intein C-fragment degron of the invention, from the N to the C-terminus would be:
(Degron)- (linker) - (Intein-C fragment) - (C-terminal portion of the protein to be reconstituted)

Preferably, the Intein C of any of **the split intein C-fragment of the invention** or **the split intein C-fragment degron of the invention** is selected from any of the following listed in table 4 below:

**Table 4**

| | | |
|---|---|---|
| **Cfa^{c}** | MVKIISRKSLGTQNVYDIGVEKDHNFLLKNGLVASN | 28 |
| **Cfa^{Cmut}** | MVKIISRKSLGTQNVYDIGVGEPHNFLLKNGLVASN | 29 |
| **Cat^{C}** | MFKLNTKNIKVLTPSGFKSFSGIQKVYKPFYHHIIFD DGSEIKCSDNHSF GKDKIKASTIKVGDYLQGKKVLYNEIVEEGIYLYDLL NVGEDNLYYTNGI VSHN | 31 |
| **Npu^{C}** | MIKIATRKYLGKQNVYDIGVERDHNFALKNGFIASN | 33 |
| **Npu^{Cmut}** | MIKIATRKYLGKQNVYDIGVGEPHNFALKNGFIASN | 36 |

wherein preferably the Intein C is the CfaC intein of SEQ ID NO 28 or any of its variants such as SEQ ID NO 29, or the Cat intein (SEQ ID NO 31), or any Cat or Cfa variant with increased promiscuity. Variants of the Intein C include SEQ ID NO 28, 29, 31, 33 and 36 without the N-terminal Methionine residue.

In a particular embodiment, the split intein C-fragment comprises or consists of a variant of the amino acid sequence of SEQ ID NO: 28 having at least 90% sequence identity with SEQ ID NO: 28 over the whole sequence.

In a particular embodiment, the split intein C-fragment comprises or consists of a variant of the amino acid sequence of SEQ ID NO: 31 having at least 90% sequence identity with SEQ ID NO: 31 over the whole sequence.

The variant of the split intein N-fragment of SEQ ID NO: 28 has at least 90% sequence identity with SEQ ID NO: 28. In a particular embodiment, the variant of the split intein N-fragment of SEQ ID NO: 28 has at least 91%, at least 92%, at least 93%, at least 94%, at least 95%, at least 96%, at least 97%, at least 98%, at least 99% sequence identity with SEQ ID NO: 28.

The variant of the split intein N-fragment of SEQ ID NO: 31 has at least 90% sequence identity with SEQ ID NO: 31. In a particular embodiment, the variant of the split intein N-fragment of SEQ ID NO: 31 has at least 91%, at least 92%, at least 93%, at least 94%, at least 95%, at least 96%, at least 97%, at least 98%, at least 99% sequence identity with SEQ ID NO: 31.

The term "linker" between the split intein C-fragment and the degron is the same as the linker already defined for the split intein N-fragment and the degron.

In another particular embodiment, the degron directly linked via a peptide bond to the split intein C-fragment is selected from any of those listed in table 3.

In yet another preferred embodiment, preferred combinations of split intein C-fragments and degrons are selected from the list consisting of the CfaC intein and DD1, SopE, SopE1-78, SopE15-78, or SopE15-50. In yet another preferred embodiment, preferred combinations of split intein C-fragments and degrons are selected from the list consisting of the CfaC intein and SEQ ID NO 42, 43, 49, 50, 51, 54 or 34.

### Complex comprising a split intein C-fragment

In another aspect, the invention relates to any of the following complex/es: the split intein C-fragment of the invention or the split intein C-fragment degron of the invention, hereinafter second complex/es of the invention, wherein each of these complexes comprises:
(i) the C-terminal fragment of a protein of interest, and
(ii) a split intein C-fragment (the split intein C-fragment of the invention) or a split intein C-fragment directly linked via a peptide bond to a degron, optionally through a linker, as defined in the above section (the split intein C-fragment degron of the invention);
wherein the complex/es optionally comprise a linker between (i) and (ii) and
wherein
- the C-terminal fragment of the protein of interest is linked to the C-terminus of the split intein C-fragment by an amide linkage, or
- if the complex/es comprise a linker, the C-terminal fragment of the protein of interest is bound to the linker by an amide linkage and/or the linker is bound to the C-terminus of the split intein C-fragment by an amide linkage.

Illustrative non-limitative examples of proteins of interest, useful in the present invention, are those listed in table 1 above. Further examples of proteins of interest can be selected from antibodies, antibody fragments, including Fc domain, scFv, nanobodies, bi-specific antibodies, proteins, and, preferably, any proteins larger than 25, 50, 100 KDa.

The terms "protein of interest" and "linker" have been previously defined in connection with the first complex of the invention. All the particular embodiments of the protein of interest and linker of the first complex of the invention fully apply to the second complex of the invention.

In a particular embodiment, the complex does not comprise a linker between the compound of interest and the split intein C-fragment. In this particular embodiment, the compound of interest is linked to the C-terminus of the split intein C-fragment by an amide linkage.

In a particular embodiment, the complex comprises a linker between the compound of interest and the split intein C-fragment. In this particular embodiment, the compound of interest may be bound to the linker by any suitable means, depending on the chemical nature of the compound of interest and of the linker. In this particular embodiment, the linker is bound to the C-terminus of the split intein C-fragment by an amide linkage. In another particular embodiment, the compound of interest is bound to the linker by an amide linkage, in which case the linker may be bound to the C-terminus of the split intein C-fragment by any suitable means. In another particular embodiment, the compound of interest is bound to the linker by an amide linkage and the linker is bound to the C-terminus of the split intein C-fragment by an amide linkage.

In a particular embodiment, if the complex comprises a linker, the linker is a peptide linker. In this particular embodiment, the complex is a fusion protein.

### Composition comprising the complexes of the invention

In another aspect, the invention refers to a composition, hereinafter first composition of the invention, comprising the first **and/or** the second complex/es of the invention.

The term "composition" is intended to encompass a product containing the specified components, as well as any product that results, directly or indirectly, from a combination of the specified components in the specified amounts. The components of the composition may be packed together in a single formulation or separately in different formulations. Thus, in an embodiment, the first complex of the invention is packed together with the second complex of the invention in a single formulation. In another embodiment, the first complex of the invention and of the second complex of the invention are separately packed.

In a particularly preferred embodiment, the first and the second complex comprise the N-terminal fragment and the C-terminal fragment of the same protein respectively, in such a way that when both complexes are combined according to the methods of the invention, the N-terminal fragment of the protein is linked to the C-terminal fragment of the protein generating the whole protein.

### Polynucleotides, vectors and host cells of the invention

In another aspect, the invention relates to polynucleotides encoding the first, or second complex/es of the invention.

Preferably, in a preferred embodiment, the invention refers to two polynucleotides, wherein one of these polynucleotides encodes the first complex/es of the invention (hereinafter first polynucleotide of the invention), and the other encodes the second complex/es of the invention (hereinafter second polynucleotide of the invention), wherein the first and the second polynucleotide of the invention encode the N-terminal fragment and the C-terminal fragment of the same protein respectively, in such a way that when both polynucleotides are translated into their respective protein complexes and combined according to the methods of the invention, the N-terminal fragment of the protein is linked to the C-terminal fragment of the protein thus generating a the whole protein to be reconstituted. It is important to note that the first and second polynucleotides of the invention each preferably encodes split inteins of the same intein. However, independently of the fact of whether both polynucleotides encodes split inteins pertaining to the same intein or not, once translated into a protein, the N-terminal and C-terminal sequences of each of the split inteins must become separate fragments that can non-covalently re-associate, or reconstitute, into an intein that is functional for trans-splicing reactions.

In another preferred embodiment, the invention refers to two polynucleotides, wherein one of these polynucleotides encodes the split intein N-fragment degron of the invention (hereinafter first polynucleotide encoding degron of the invention), and the other encodes the split intein C-fragment degron of the invention (hereinafter second polynucleotide encoding degron of the invention), wherein the first and the second polynucleotide encoding degron of the invention encode the N-terminal fragment and the C-terminal fragment of the same protein respectively, in such a way that when both polynucleotides are translated into their respective protein complexes and combined according to the methods of the invention, the N-terminal fragment of the protein is linked to the C-terminal fragment of the protein thus generating the whole protein. As with the previous embodiment, it is important to note that the first and second polynucleotides encoding degron of the invention each preferably encode split inteins of the same intein. At any rate and as reflected above, as used herein each polynucleotide must encode a split intein so that, once translated into a protein, the N-terminal and C-terminal sequences become separate fragments that can non-covalently re-associate, or reconstitute, into an intein that is functional for trans-splicing reactions.

In yet another preferred embodiment, the invention refers to a three-piece ligation strategy using orthogonal split intein pairs. In this methodology, there are three polynucleotides, wherein the first polynucleotide encodes the POI^{N}-Cfa^{N} (wherein POI^{N} is understood as the N fragment of a "protein of interest); wherein the second polynucleotide encodes the Cfa^{C}-POI^{M}-Cat^{N} (wherein the POI^{M} in this case, is an intermediate fragment of a protein of interest); and wherein the third polynucleotide encodes the Cat^{C}-POI^{C} (wherein POI^{C} is understood as the C fragment of a protein of interest).. Alternatively, the positions of the Cfa and Cat inteins could be swapped, to generate constructs with the following architecture: POI^{N}-Cat^{N}, Cat^{C}-POI^{M}-Cfa^{N} and Cfa^{C}-POI^{C} CfaN is the CfaN intein fragment SEQ ID NO 27, as well as any of its variants. CatN is the CatN intein fragment SEQ ID NO 30 as well as any of its variants. The CfaC is the CfaC intein fragment SEQ ID NO 28, as well as any of its variants, including SEQ ID NO 29. The CatC is the CatC intein fragment SEQ ID NO 31, as well as any of its variants,

It is herein noted that for those embodiments referring to two polynucleotides, the split intein N-fragments of the invention, or the split intein-N degron fragment of the invention, coded by any of the above-mentioned polynucleotides (first polynucleotide of the invention) can be selected from any of the following list consisting of: SEQ ID NO 1,3, 5, 8, 10, 12, 14, 17, 19, 21, 23, 24, 25, 66, 68, 70, 72, 74, 76 and 78. It is herein noted that for those embodiments referring to two polynucleotides, the split intein C-fragments of the invention or the split intein-C degron fragment of the invention, coded by any of the above-mentioned polynucleotides (second polynucleotide of the invention) can be selected from any of following list consisting of: SEQ ID NO 2, 4, 6, 9, 11, 13, 15, 18, 20, 22, 26, 67, 69, 71, 73, 75, 77 and 79.

Preferred combinations of nucleotide sequences coding for the above sequences (respectively for the first and second polynucleotides of the invention) are selected from any of the following pairs: 1 and 2; 3 and 4; 5 and 6; 66 and 67; 68 and 69; 70 and 71; 72 and 73; 74 and 75; 76 and 77; and 78 and 79.

Preferably, the invention refers to a composition, hereinafter second composition of the invention, comprising the first and/or the second polynucleotide of the invention or the first and/or the second polynucleotide encoding degron of the invention and/or separately or jointly each of the POI-intein fragments of the three-piece ligation system using orthogonal split intein pairs. The term "composition", in this specific context, is intended to encompass a product containing the specified components. The components of the composition may be packed together in a single formulation or separately in different formulations. Thus, in an embodiment, the first polynucleotide is packed together with the second polynucleotide in a single formulation. In another embodiment, the first polynucleotide and the second polynucleotide are separately packed. More preferably, the first and the second polynucleotides, respectively, encode the N-terminal fragment and the C-terminal fragment of the protein to be reconstituted, in such a way that when both complexes are combined according to the methods of the invention, the N-terminal fragment of the protein is linked to the C-terminal fragment of the protein generating the whole protein to be reconstituted.

All of the above-referred polynucleotides of the invention can be found isolated as such or forming part of vectors allowing the propagation of said polynucleotides in suitable host cells. Therefore, in another aspect, the invention relates to a vector comprising the polynucleotide of the invention as described above.

Vectors suitable for the insertion of said polynucleotide are vectors derived from expression vectors in prokaryotes such as pUC18, pUC19, Bluescript and the derivatives thereof, mpl8, mpl9, pBR322, pMB9, ColEl, pCRI, RP4, phages and "shuttle" vectors such as pSA3 and pAT28; expression vectors in yeasts such as vectors of the type of 2 micron plasmids, integration plasmids, YEP vectors, centromere plasmids and the like; expression vectors in insect cells such as vectors of the pAC series and of the pVL; expression vectors in plants such as pIBI, pEarleyGate, pAVA, pCAMBIA, pGSA, pGWB, pMDC, pMY, pORE series and the like; and expression vectors in eukaryotic cells, including baculovirus suitable for transfecting insect cells using any commercially available baculovirus system. The vectors for eukaryotic cells include preferably viral vectors (adenoviruses, adeno associated viruses (AAV) viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAXl, pZeoSV2, pCI, pSVL and PKSV-10, pBPV-1, pML2d and pTDTl . Preferably, the vectors are adeno associated viruses (AAV). Preferably the vectors are AAVs of serotype 1, 2, 3, 4, 5, 6, 7, 8, or 9. Dimeric, or self-complementary AAV vectors (scAAV), could also be used for the insertion of said polynucleotides.

The present invention is thus preferably directed to development of AAVs as gene therapy vectors. Preferably, these vectors have eliminated their integrative capacity by removal of the rep and cap from the DNA of the vector. The desired gene (polynucleotide) together with a promoter to drive transcription of the gene is inserted between the inverted terminal repeats (ITR) that aid in concatamer formation in the nucleus after the single-stranded vector DNA is converted by host cell DNA polymerase complexes into double-stranded DNA. AAV-based gene therapy vectors form episomal concatamers in the host cell nucleus. In non-dividing cells, these concatemers remain intact for the life of the host cell. In dividing cells, AAV DNA is lost through cell division, since the episomal DNA is not replicated along with the host cell DNA. Random integration of AAV DNA into the host genome is detectable but occurs at very low frequency.

The desired gene (polynucleotide) might be combined with regulatory elements that are functional in the intended host cell in which the construct is to be expressed. A person of ordinary skill in the art can select regulatory elements for use in appropriate host cells, for example, mammalian or human host cells. Regulatory elements include, for example, promoters, transcription termination sequences, translation termination sequences, enhancers, signal peptides, and polyadenylation elements. The polynucleotides of the invention can be operably linked to a promoter sequence. Promoters contemplated for use in the subject invention include, but are not limited to, native gene promoters, cytomegalovirus (CMV) promoter (KF853603.1, bp 149-735), chimeric CMV/chicken beta-actin promoter (CBA) and the truncated form of CBA (smCBA) promoter (US8298818 and Light-Driven Cone Arrestin Translocation in Cones of Postnatal Guanylate Cyclase- 1 Knockout Mouse Retina Treated with AAVGC 1), Rhodopsin promoter (NG 009115, bp 4205-5010), Interphotoreceptor retinoid binding protein (IRBP) promoter (NG_029718.1, bp 4777- 5011), vitelliform macular dystrophy 2 (VMD2) promoter (NG 009033.1, bp 4870-5470), PR-specific human G protein-coupled receptor kinase 1 (hGRKI; AY327580.1 bpl793-2087 or bp 1793- 1991) (Haire et al. 2006; U.S. Patent No. 8,298,818), proximal murine rhodopsin promoter (MOPS). However any suitable promoter known in the art may be used. In a specific embodiment, the promoter is a CMV or hGRKl promoter. In one embodiment, the promoter is a tissue- specific promoter that shows selective activity in one or a group of tissues but is less active or not active in other tissue. In one embodiment, the promoter is a photoreceptor- specific promoter. In a further embodiment, the promoter is a cone cell-specific and/or rod cell-specific promoter.

Preferred promoters are CMV, GRK1, CBA and IRBP promoters. Still preferred promoters are hybrid promoter which combine regulatory elements from various promoters (as example the chimeric CBA promoter which combines an enhancer from the CMV promoter, the CBA promoter and the Sv40 chimeric intron, herein called CBA hybrid promoter.

AAVs also present very low immunogenicity, seemingly restricted to generation of neutralizing antibodies, while they induce no clearly defined cytotoxic response. This feature, along with the ability to infect quiescent cells present their dominance over adenoviruses as vectors for human gene therapy.

AAV genome, transcriptome and proteome: The AAV genome is built of single-stranded deoxyribonucleic acid (ssDNA), either positive-or negative-sensed, which is about 4.7 kilobase long. The genome comprises inverted terminal repeats (ITRs) at both ends of the DNA strand, and two open reading frames (ORFs): rep and cap. The former is composed of four overlapping genes encoding Rep proteins required for the AAV life cycle, and the latter contains overlapping nucleotide sequences of capsid proteins: VP1, VP2 and VP3, which interact together to form a capsid of an icosahedral symmetry.
ITR sequences The Inverted Terminal Repeat (ITR) sequences comprise 145 bases each. They were named so because of their symmetry, which was shown to be required for efficient multiplication of the AAV genome. Another property of these sequences is their ability to form a hairpin, which contributes to so-called self-priming that allows primase-independent synthesis of the second DNA strand. The ITRs were also shown to be required for both integration of the AAV DNA into the host cell genome (19th chromosome in humans) and rescue from it, as well as for efficient encapsidation of the AAV DNA combined with generation of a fully assembled, deoxyribonuclease-resistant AAV particles.

With regard to gene therapy, ITRs seem to be the only sequences required in cis next to the therapeutic gene: structural (cap) and packaging (rep) genes can be delivered in trans. With this assumption many methods were established for efficient production of recombinant AAV
(rAAV) vectors containing a reporter or therapeutic gene. However, it was also published that the ITRs are not the only elements required in cis for the effective replication and encapsidation. A few research groups have identified a sequence designated cis-acting Rep-dependent element (CARE) inside the coding sequence of the rep gene. CARE was shown to augment the replication and encapsidation when present in cis.

As of 2006 there have been 11 AAV serotypes described. All of the known serotypes can infect cells from multiple diverse tissue types. Tissue specificity is determined by the capsid serotype and pseudotyping of AAV vectors to alter their tropism range will likely be important to their use in therapy. In the present invention ITRs of AVV serotype 1, 2, 3, 4, 5, 6, 7, 8, or 9 are prefered. Serotype 2 Serotype 2 (AAV2) has been the most extensively examined so far. AAV2 presents natural tropism towards skeletal muscles, neurons, vascular smooth muscle cells and hepatocytes.

The vectors may also comprise a reporter or marker gene which allows identifying those cells that have incorporated the vector after having been put in contact with it.

Useful reporter genes in the context of the present invention include lacZ, luciferase, thymidine kinase, GFP and on the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolatereductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of E. coli, allowing the cells to grow in the presence of indol instead of tryptophan; the hisD gene of E. coli, allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

On the other hand, as the skilled person in the art knows, the choice of the vector will depend on the host cell in which it will subsequently be introduced. By way of example, the vector in which said polynucleotide is introduced can also be a yeast artificial chromosome (YAC), a bacterial artificial chromosome (BAC) or a PI -derived artificial chromosome (PAC). The characteristics of the YAC, BAC and PAC are known by the person skilled in the art. Detailed information on said types of vectors has been provided, for example, by Giraldo and Montoliu (Giraldo, P. & Montoliu L., 2001 Size matters: use of YACs, BACs and PACs in transgenic animals, Transgenic Research 10(2): 83-110). The vector of the invention can be obtained by conventional methods known by persons skilled in the art (Sambrook J. et al., 2000 "Molecular cloning, a Laboratory Manual", 3rd ed., Cold Spring Harbor Laboratory Press, N.Y. Vol 1-3).

The polynucleotide of the invention can be introduced into the host cell in vivo as naked DNA plasmids, but also using vectors by methods known in the art, including but not limited to transfection, electroporation (e.g. transcutaneous electroporation), microinjection, transduction, cell fusion, DEAE dextran, calcium phosphate precipitation, use of a gene gun, or use of a DNA vector transporter. Methods for formulating and administering naked DNA to mammalian muscle tissue are also known. See Feigner P, et al., US 5,580,859, and US 5,589,466. Other molecules are also useful for facilitating transfection of a nucleic acid in vivo, such as cationic oligopeptides, peptides derived from DNA binding proteins, or cationic polymers. See Bazile D, et al., WO 1995021931, and Byk G, et al., WO 1996025508.

Another well-known method that can be used to introduce polynucleotides into host cells is particle bombardment (aka biolistic transformation). Biolistic transformation is commonly accomplished in one of several ways. One common method involves propelling inert or biologically active particles at cells. See Sanford J, et al., US 4,945,050, US 5,036,006, and US 5,100,792.

Alternatively, the vector can be introduced *in vivo* by lipofection. The use of cationic lipids can promote encapsulation of negatively charged nucleic acids, and also promote fusion with negatively charged cell membranes. See Feigner P, Ringold G, Science 1989; 337:387-388. Particularly useful lipid compounds and compositions for transfer of nucleic acids have been described. See Feigner P, et al., US 5,459,127, Behr J, et al., WO1995018863, and Byk G, WO1996017823.

Finally, and specially preferred, the vector can be introduced *in vivo* by viral delivery systems including but not limited to adenoviral vectors, adeno-associated viral (AAV) vectors, pseudotyped AAV vectors, herpes viral vectors, retroviral vectors, lentiviral vectors, baculoviral vectors. Pseudotyped AAV vectors are those which contain the genome of one AAV serotype in the capsid of a second AAV serotype; for example an AAV2/8 vector contains the AAV8 capsid and the AAV 2 genome (Auricchio et al. (2001) Hum. Mol. Genet. 10(26):3075-81). Such vectors are also known as chimeric vectors. Other examples of delivery systems include ex vivo delivery systems, which include but are not limited to DNA transfection methods such as electroporation, DNA biolistics, lipid-mediated transfection, compacted DNA-mediated transfection.

The construction of an AAV vector can be carried out following procedures and using techniques which are known to a person skilled in the art. The theory and practice for adeno-associated viral vector construction and use in therapy are illustrated in several scientific and patent publications (the following bibliography is herein incorporated by reference: Flotte TR. Adeno-associated virus-based gene therapy for inherited disorders. Pediatr Res. 2005 Dec;58(6):1143-7; Goncalves MA. Adeno-associated virus: from defective virus to effective vector, Virol J. 2005 May 6;2:43; Surace EM, Auricchio A. Adeno-associated viral vectors for retinal gene transfer. Prog Retin Eye Res. 2003 Nov;22(6):705-19; Mandel RJ, Manfredsson FP, Foust KD, Rising A, Reimsnider S, Nash K, Burger C. Recombinant adeno-associated viral vectors as therapeutic agents to treat neurological disorders. Mol Ther. 2006 Mar;13(3):463-83).

Suitable administration forms of a pharmaceutical composition containing AAV vectors include, but are not limited to, injectable solutions or suspensions, eye lotions and ophthalmic ointment. Thus, in another aspect, the invention relates to a host cell comprising the polynucleotide or the vector of the invention. The cells can be obtained by conventional methods known by persons skilled in the art (see e.g. Sambrook et al., cited ad supra).

The term "host cell", as used herein, refers to a cell into which a nucleic acid of the invention, such as a polynucleotide or a vector according to the invention, has been introduced and is capable of expressing the split intein N-fragment of the invention or the fusion protein comprising said split intein N-fragment. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact be identical to the parent cell, but are still included within the scope of the term as used herein. The term includes any cultivatable cell that can be modified by the introduction of heterologous DNA. Preferably, a host cell is one in which the polynucleotide of the invention can be stably expressed, post-translationally modified, localized to the appropriate subcellular compartment, and made to engage the appropriate transcription machinery. The choice of an appropriate host cell will also be influenced by the choice of detection signal. For example, reporter constructs, as described above, can provide a selectable or screenable trait upon activation or inhibition of gene transcription in response to a transcriptional regulatory protein; in order to achieve optimal selection or screening, the host cell phenotype will be considered. A host cell of the present invention includes prokaryotic cells and eukaryotic cells. Prokaryotes include gram negative or gram positive organisms, for example, E. coli or Bacilli. It is to be understood that prokaryotic cells will be used, preferably, for the propagation of the transcription control sequence comprising polynucleotides or the vector of the present invention. Suitable prokaryotic host cells for transformation include, for example, E. coli, Bacillus subtilis, Salmonella typhimurium, and various other species within the genera Pseudomonas, Streptomyces, and Staphylococcus. Eukaryotic cells include, but are not limited to, yeast cells, plant cells, fungal cells, insect cells (e.g., baculovirus), mammalian cells, and the cells of parasitic organisms, e.g., trypanosomes. As used herein, yeast includes not only yeast in a strict taxonomic sense, i.e., unicellular organisms, but also yeast-like multicellular fungi of filamentous fungi. Exemplary species include Kluyverei lactis, Schizosaccharomyces pombe, and Ustilaqo maydis, with Saccharomyces cerevisiae being preferred. Other yeasts which can be used in practicing the present invention are Neurospora crassa, Aspergillus niger, Aspergillus nidulans, Pichia pastoris, Candida tropicalis, and Hansenula polymorpha. Mammalian host cell culture systems include established cell lines such as COS cells, L cells, 3T3 cells, Chinese hamster ovary (CHO) cells, embryonic stem cells, with BHK, HeK or HeLa cells being preferred. Eukaryotic cells are, preferably, used for recombinant gene expression.

In a preferred embodiment, the second composition of the invention is for use in therapy, more particularly, for use in any of the diseases identified in table 1 depending on the type of gene coded for in the composition (a correlation between disease and gene is clearly indicated in table 1, and it is thus evident for the skilled person to identified the correct combinations).

### Methods for expressing a gene encoding a protein of interest in a cell

In another aspect, the invention relates to a method for expressing a gene of interest in a cell, hereinafter first method for expressing a gene of interest, comprising:
(i) contacting the cell with
   (a) a first polynucleotide of the invention or a first polynucleotide encoding degron of the invention, and
   (b) a second polynucleotide of the invention or a second polynucleotide encoding degron of the invention,
(ii) allowing the expression of the first and/or the second polynucleotides so that the first and the second fusion proteins are produced and
(iii) allowing the contact between the first and second proteins so that the split intein N-fragment binds to the split intein C-fragment to form an intein intermediate and the intein intermediate reacts to covalently link the C-terminus of the first polypeptide of interest to the N-terminus of the second polypeptide of interest.

In a particular embodiment, the first polynucleotide is the first polynucleotide encoding degron and the second polynucleotide is the second polynucleotide encoding degron, wherein, preferably, the protein of interest is of more than 25 KDa, more than 50 KDa or more than 100 KDa, so that upon covalently linking the C-terminus of the first polypeptide of interest to the N-terminus of the second polypeptide of interest the whole protein is obtained.

The contacting of the cell with the first and/or second polynucleotide can be made by any suitable means for allowing introducing a polynucleotide of interest into a cell, for example, transfection, electroporation, microinjection, transduction, lipofection, cell fusion, DEAE dextran, calcium phosphate precipitation, use of a gene gun, **or use of a DNA vector transporter.** Preferably, the vectors are adeno associated viruses (AAV).

In a preferred embodiment of the invention, relates to a method for expressing a gene encoding a protein of interest in a cell, hereinafter fist method for expressing a gene of interest, comprising:
(i) contacting, or transducing, the cell with
   (a) **a first AAV comprising** a first polynucleotide of the invention or a first polynucleotide encoding degron of the invention, and
   (b) a **second AAV comprising** a second polynucleotide of the invention or a second polynucleotide encoding degron of the invention,
(ii) allowing the expression of the first and the second polynucleotides so that the first and the second fusion proteins are produced and
(iii) allowing the contact between the first and second fusion proteins so that the split intein N-fragment binds to the split intein C-fragment to form an intein intermediate and the intein intermediate reacts to covalently link the C-terminus of the first polypeptide of interest to the N-terminus of the second polypeptide of interest,
wherein preferably, the first polynucleotide is the first polynucleotide encoding degron and the second polynucleotide is the second polynucleotide encoding degron, and wherein, more preferably, the protein of interest is of more than 25 KDa, more than 50 KDa or more than 100 KDa, so that upon covalently linking the C-terminus of the first polypeptide of interest to the N-terminus of the second polypeptide of interest the whole protein is obtained.

In this sense and as illustrated in the examples, the presence of degrons will cause the fast degradation of starting materials. Selected degrons have kinetics of degradation compatible with protein splicing in such a manner, that in the steady state the amount of starting material is reduced relative to what is observed in the absence of degrons. But the levels of splicing product are maintained, or increase, relative to what is observed in the absence of degrons.

In the first method for expressing a gene of interest of the invention, it is contemplated that the cell is contacted simultaneously with the first and second polynucleotide, or sequentially with the first and second polynucleotide in any order, that is, the cell can be contacted firstly with the first polynucleotide and secondly with the second polynucleotide or firstly with the second polynucleotide and secondly with the first polynucleotide. The same should hold true with the vectors encoding said polynucleotides.

Any cell previously defined as a host cell can be used in these methods.

The invention will be described by way of the following examples which are to be considered as merely illustrative and not limitative of the scope of the invention.

### Examples

### List of sequences used throughout the examples:

| **Construct** | **^{a}Sequence** | **SEQ NO** |
|---|---|---|
| **ABCA4-1150-Cfa^{N}**-**3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYDTEILTVEYGFLPIGKIVEERIECTVYTVDK NGFVYTQPIAQWHNRGEQEVFEYCLEDGSIIRATKDH KFMTTDGQMLPIDEIFERGLDLKQVDGLPDYKDHDGD YKDHDIDYKDDDDK | 1 |
| **ABCA4-1150-Cfa^{C}**-**3FT** | MVKIISRKSLGTQNVYDIGVEKDHNFLLKNGLVASNC FGTGLYLTLVRKMKNIQSQRKGSEGTCSCSSKGFSTT CPAHVDDLTPEQVLDGDVNELMDVVLHHVPEAKLVEC IGQELIFLLPNKNFKHRAYASLFRELEETLADLGLSS FGISDTPLEEIFLKVTEDSDSGPLFAGGAQQKRENVN PRHPCLGPREKAGQTPQDSNVCSPGAPAAHPEGQPPP EPECPGPQLNTGTQLVLQHVQALLVKRFQHTIRSHKD FLAQIVLPATFVFLALMLSIVIPPFGEYPALTLHPWI YGQQYTFFSMDEPGSEQFTVLADVLLNKPGFGNRCLK EGWLPEYPCGNSTPWKTPSVSPNITQLFQKQKWTQVN PSPSCRCSTREKLTMLPECPEGAGGLPPPQRTQRSTE ILQDLTDRNISDFLVKTYPALIRSSLKSKFWVNEQRY GGISIGGKLPVVPITGEALVGFLSDLGRIMNVSGGPI TREASKEIPDFLKHLETEDNIKVWFNNKGWHALVSFL | 2 |
| | NVAHNAILRASLPKDRSPEEYGITVISQPLNLTKEQL SEITVLTTSVDAVVAICVIFSMSFVPASFVLYLIQER VNKSKHLQFISGVSPTTYWVTNFLWDIMNYSVSAGLV VGIFIGFQKKAYTSPENLPALVALLLLYGWAVIPMMY PASFLFDVPSTAYVALSCANLFIGINSSAITFILELF ENNRTLLRFNAVLRKLLIVFPHFCLGRGLIDLALSQA VTDVYARFGEEHSANPFHWDLIGKNLFAMVVEGVVYF LLTLLVQRHFFLSQWIAEPTKEPIVDEDDDVAEERQR IITGGNKTDILRLHELTKIYPGTSSPAVDRLCVGVRP GECFGLLGVNGAGKTTTFKMLTGDTTVTSGDATVAGK SILTNISEVHQNMGYCPQFDAIDELLTGREHLYLYAR LRGVPAEEIEKVANWSIKSLGLTVYADCLAGTYSGGN KRKLSTAIALIGCPPLVLLDEPTTGMDPQARRMLWNV IVSIIREGRAVVLTSHSMEECEALCTRLAIMVKGAFR CMGTIQHLKSKFGDGYIVTMKIKSPKDDLLPDLNPVE QFFQGNFPGSVQRERHYNMLQFQVSSSSLARIFQLLL SHKDSLLIEEYSVTQTTLDQVFVNFAKQQTESHDLPL HPRAAGASRQAQDDYKDHDGDYKDHDIDYKDDDDK | |
| **ABCA4-1140-Cfa^{N}**-**3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCLSYDTEI LTVEYGFLPIGKIVEERIECTVYTVDKNGFVYTQPIA QWHNRGEQEVFEYCLEDGSIIRATKDHKFMTTDGQML PIDEIFERGLDLKQVDGLPDYKDHDGDYKDHDIDYKD DDDK | 3 |
| **ABCA4-1140-Cfa^{Cmut}-3FT** | MVKIISRKSLGTQNVYDIGVGEPHNFLLKNGLVASNC SGTPLFLKNCFGTGLYLTLVRKMKNIQSQRKGSEGTC SCSSKGFSTTCPAHVDDLTPEQVLDGDVNELMDVVLH HVPEAKLVECIGQELIFLLPNKNFKHRAYASLFRELE | 4 |
| | ETLADLGLSSFGISDTPLEEIFLKVTEDSDSGPLFAG GAQQKRENVNPRHPCLGPREKAGQTPQDSNVCSPGAP AAHPEGQPPPEPECPGPQLNTGTQLVLQHVQALLVKR FQHTIRSHKDFLAQIVLPATFVFLALMLSIVIPPFGE YPALTLHPWIYGQQYTFFSMDEPGSEQFTVLADVLLN KPGFGNRCLKEGWLPEYPCGNSTPWKTPSVSPNITQL FQKQKWTQVNPSPSCRCSTREKLTMLPECPEGAGGLP PPQRTQRSTEILQDLTDRNISDFLVKTYPALIRSSLK SKFWVNEQRYGGISIGGKLPVVPITGEALVGFLSDLG RIMNVSGGPITREASKEIPDFLKHLETEDNIKVWFNN KGWHALVSFLNVAHNAILRASLPKDRSPEEYGITVIS QPLNLTKEQLSEITVLTTSVDAVVAICVIFSMSFVPA SFVLYLIQERVNKSKHLQFISGVSPTTYWVTNFLWDI MNYSVSAGLVVGIFIGFQKKAYTSPENLPALVALLLL YGWAVIPMMYPASFLFDVPSTAYVALSCANLFIGINS SAITFILELFENNRTLLRFNAVLRKLLIVFPHFCLGR GLIDLALSQAVTDVYARFGEEHSANPFHWDLIGKNLF AMVVEGVVYFLLTLLVQRHFFLSQWIAEPTKEPIVDE DDDVAEERQRIITGGNKTDILRLHELTKIYPGTSSPA VDRLCVGVRPGECFGLLGVNGAGKTTTFKMLTGDTTV TSGDATVAGKSILTNISEVHQNMGYCPQFDAIDELLT GREHLYLYARLRGVPAEEIEKVANWSIKSLGLTVYAD CLAGTYSGGNKRKLSTAIALIGCPPLVLLDEPTTGMD PQARRMLWNVIVSIIREGRAVVLTSHSMEECEALCTR LAIMVKGAFRCMGTIQHLKSKFGDGYIVTMKIKSPKD DLLPDLNPVEQFFQGNFPGSVQRERHYNMLQFQVSSS SLARIFQLLLSHKDSLLIEEYSVTQTTLDQVFVNFAK QQTESHDLPLHPRAAGASRQAQDDYKDHDGDYKDHDI DYKDDDDK | |
| **ABCA4-1188-Cfa^{N}**-**3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE | 5 |
| | EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCFGTGLYLTLVRKMKNIQSQRKGSEGTCSCSSKGF STTCLSYDTEILTVEYGFLPIGKIVEERIECTVYTVD KNGFVYTQPIAQWHNRGEQEVFEYCLEDGSIIRATKD HKFMTTDGQMLPIDEIFERGLDLKQVDGLPDYKDHDG DYKDHDIDYKDDDDK | |
| **ABCA4-1188-Cfa^{cmut}**-**3FT** | MVKIISRKSLGTQNVYDIGVGEPHNFLLKNGLVASNC PAHVDDLTPEQVLDGDVNELMDVVLHHVPEAKLVECI GQELIFLLPNKNFKHRAYASLFRELEETLADLGLSSF GISDTPLEEIFLKVTEDSDSGPLFAGGAQQKRENVNP RHPCLGPREKAGQTPQDSNVCSPGAPAAHPEGQPPPE PECPGPQLNTGTQLVLQHVQALLVKRFQHTIRSHKDF LAQIVLPATFVFLALMLSIVIPPFGEYPALTLHPWIY GQQYTFFSMDEPGSEQFTVLADVLLNKPGFGNRCLKE GWLPEYPCGNSTPWKTPSVSPNITQLFQKQKWTQVNP SPSCRCSTREKLTMLPECPEGAGGLPPPQRTQRSTEI LQDLTDRNISDFLVKTYPALIRSSLKSKFWVNEQRYG GISIGGKLPVVPITGEALVGFLSDLGRIMNVSGGPIT REASKEIPDFLKHLETEDNIKVWFNNKGWHALVSFLN VAHNAILRASLPKDRSPEEYGITVISQPLNLTKEQLS EITVLTTSVDAVVAICVIFSMSFVPASFVLYLIQERV NKSKHLQFISGVSPTTYWVTNFLWDIMNYSVSAGLVV GIFIGFQKKAYTSPENLPALVALLLLYGWAVIPMMYP ASFLFDVPSTAYVALSCANLFIGINSSAITFILELFE NNRTLLRFNAVLRKLLIVFPHFCLGRGLIDLALSQAV TDVYARFGEEHSANPFHWDLIGKNLFAMVVEGVVYFL LTLLVQRHFFLSQWIAEPTKEPIVDEDDDVAEERQRI ITGGNKTDILRLHELTKIYPGTSSPAVDRLCVGVRPG ECFGLLGVNGAGKTTTFKMLTGDTTVTSGDATVAGKS ILTNISEVHQNMGYCPQFDAIDELLTGREHLYLYARL RGVPAEEIEKVANWSIKSLGLTVYADCLAGTYSGGNK RKLSTAIALIGCPPLVLLDEPTTGMDPQARRMLWNVI VSIIREGRAVVLTSHSMEECEALCTRLAIMVKGAFRC MGTIQHLKSKFGDGYIVTMKIKSPKDDLLPDLNPVEQ FFQGNFPGSVQRERHYNMLQFQVSSSSLARIFQLLLS HKDSLLIEEYSVTQTTLDQVFVNFAKQQTESHDLPLH PRAAGASRQAQDDYKDHDGDYKDHDIDYKDDDDK | 6 |
| **ABCA4-3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS | 7 |
| | VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCFGTGLYLTLVRKMKNIQSQRKGSEGTCSCSSKGF STTCPAHVDDLTPEQVLDGDVNELMDVVLHHVPEAKL VECIGQELIFLLPNKNFKHRAYASLFRELEETLADLG LSSFGISDTPLEEIFLKVTEDSDSGPLFAGGAQQKRE NVNPRHPCLGPREKAGQTPQDSNVCSPGAPAAHPEGQ PPPEPECPGPQLNTGTQLVLQHVQALLVKRFQHTIRS HKDFLAQIVLPATFVFLALMLSIVIPPFGEYPALTLH PWIYGQQYTFFSMDEPGSEQFTVLADVLLNKPGFGNR CLKEGWLPEYPCGNSTPWKTPSVSPNITQLFQKQKWT QVNPSPSCRCSTREKLTMLPECPEGAGGLPPPQRTQR STEILQDLTDRNISDFLVKTYPALIRSSLKSKFWVNE QRYGGISIGGKLPVVPITGEALVGFLSDLGRIMNVSG GPITREASKEIPDFLKHLETEDNIKVWFNNKGWHALV SFLNVAHNAILRASLPKDRSPEEYGITVISQPLNLTK EQLSEITVLTTSVDAVVAICVIFSMSFVPASFVLYLI QERVNKSKHLQFISGVSPTTYWVTNFLWDIMNYSVSA GLVVGIFIGFQKKAYTSPENLPALVALLLLYGWAVIP MMYPASFLFDVPSTAYVALSCANLFIGINSSAITFIL ELFENNRTLLRFNAVLRKLLIVFPHFCLGRGLIDLAL SQAVTDVYARFGEEHSANPFHWDLIGKNLFAMVVEGV VYFLLTLLVQRHFFLSQWIAEPTKEPIVDEDDDVAEE RQRIITGGNKTDILRLHELTKIYPGTSSPAVDRLCVG VRPGECFGLLGVNGAGKTTTFKMLTGDTTVTSGDATV AGKSILTNISEVHQNMGYCPQFDAIDELLTGREHLYL YARLRGVPAEEIEKVANWSIKSLGLTVYADCLAGTYS GGNKRKLSTAIALIGCPPLVLLDEPTTGMDPQARRML WNVIVSIIREGRAVVLTSHSMEECEALCTRLAIMVKG AFRCMGTIQHLKSKFGDGYIVTMKIKSPKDDLLPDLN PVEQFFQGNFPGSVQRERHYNMLQFQVSSSSLARIFQ LLLSHKDSLLIEEYSVTQTTLDQVFVNFAKQQTESHD LPLHPRAAGASRQAQDDYKDHDGDYKDHDIDYKDDDD K | |
| **ABCA4-1150-Npu^{N}**-**3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN | 8 |
| | STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYETEILTVEYGLLPIGKIVEKRIECTVYSVDN NGNIYTQPVAQWHDRGEQEVFEYCLEDGSLIRATKDH KFMTVDGQMLPIDEIFERELDLMRVDNLPNDYKDHDG DYKDHDIDYKDDDDK | |
| **ABCA4-1150-Npu^{C}**-**3FT** | MIKIATRKYLGKQNVYDIGVERDHNFALKNGFIASNC FGTGLYLTLVRKMKNIQSQRKGSEGTCSCSSKGFSTT CPAHVDDLTPEQVLDGDVNELMDVVLHHVPEAKLVEC IGQELIFLLPNKNFKHRAYASLFRELEETLADLGLSS FGISDTPLEEIFLKVTEDSDSGPLFAGGAQQKRENVN PRHPCLGPREKAGQTPQDSNVCSPGAPAAHPEGQPPP EPECPGPQLNTGTQLVLQHVQALLVKRFQHTIRSHKD FLAQIVLPATFVFLALMLSIVIPPFGEYPALTLHPWI YGQQYTFFSMDEPGSEQFTVLADVLLNKPGFGNRCLK EGWLPEYPCGNSTPWKTPSVSPNITQLFQKQKWTQVN PSPSCRCSTREKLTMLPECPEGAGGLPPPQRTQRSTE ILQDLTDRNISDFLVKTYPALIRSSLKSKFWVNEQRY GGISIGGKLPVVPITGEALVGFLSDLGRIMNVSGGPI TREASKEIPDFLKHLETEDNIKVWFNNKGWHALVSFL NVAHNAILRASLPKDRSPEEYGITVISQPLNLTKEQL SEITVLTTSVDAVVAICVIFSMSFVPASFVLYLIQER VNKSKHLQFISGVSPTTYWVTNFLWDIMNYSVSAGLV VGIFIGFQKKAYTSPENLPALVALLLLYGWAVIPMMY PASFLFDVPSTAYVALSCANLFIGINSSAITFILELF ENNRTLLRFNAVLRKLLIVFPHFCLGRGLIDLALSQA VTDVYARFGEEHSANPFHWDLIGKNLFAMVVEGVVYF LLTLLVQRHFFLSQWIAEPTKEPIVDEDDDVAEERQR IITGGNKTDILRLHELTKIYPGTSSPAVDRLCVGVRP GECFGLLGVNGAGKTTTFKMLTGDTTVTSGDATVAGK SILTNISEVHQNMGYCPQFDAIDELLTGREHLYLYAR LRGVPAEEIEKVANWSIKSLGLTVYADCLAGTYSGGN KRKLSTAIALIGCPPLVLLDEPTTGMDPQARRMLWNV IVSIIREGRAVVLTSHSMEECEALCTRLAIMVKGAFR CMGTIQHLKSKFGDGYIVTMKIKSPKDDLLPDLNPVE QFFQGNFPGSVQRERHYNMLQFQVSSSSLARIFQLLL SHKDSLLIEEYSVTQTTLDQVFVNFAKQQTESHDLPL HPRAAGASRQAQDDYKDHDGDYKDHDIDYKDDDDK | 9 |
| **ABCA4-1140-Npu^{N}**-**3FT** | MGFVRQIQLLLWKNWTLRKRQKIRFVVELVWPLSLFL VLIWLRNANPLYSHHECHFPNKAMPSAGMLPWLQGIF | 10 |
| | CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCLSYETEI LTVEYGLLPIGKIVEKRIECTVYSVDNNGNIYTQPVA QWHDRGEQEVFEYCLEDGSLIRATKDHKFMTVDGQML PIDEIFERELDLMRVDNLPNDYKDHDGDYKDHDIDYK DDDDK | |
| **ABCA4-1140-Npu^{C}**-**3FT** | MIKIATRKYLGKQNVYDIGVERDHNFALKNGFIASNC SGTPLFLKNCFGTGLYLTLVRKMKNIQSQRKGSEGTC SCSSKGFSTTCPAHVDDLTPEQVLDGDVNELMDVVLH HVPEAKLVECIGQELIFLLPNKNFKHRAYASLFRELE ETLADLGLSSFGISDTPLEEIFLKVTEDSDSGPLFAG GAQQKRENVNPRHPCLGPREKAGQTPQDSNVCSPGAP AAHPEGQPPPEPECPGPQLNTGTQLVLQHVQALLVKR FQHTIRSHKDFLAQIVLPATFVFLALMLSIVIPPFGE YPALTLHPWIYGQQYTFFSMDEPGSEQFTVLADVLLN KPGFGNRCLKEGWLPEYPCGNSTPWKTPSVSPNITQL FQKQKWTQVNPSPSCRCSTREKLTMLPECPEGAGGLP PPQRTQRSTEILQDLTDRNISDFLVKTYPALIRSSLK SKFWVNEQRYGGISIGGKLPVVPITGEALVGFLSDLG RIMNVSGGPITREASKEIPDFLKHLETEDNIKVWFNN KGWHALVSFLNVAHNAILRASLPKDRSPEEYGITVIS QPLNLTKEQLSEITVLTTSVDAVVAICVIFSMSFVPA SFVLYLIQERVNKSKHLQFISGVSPTTYWVTNFLWDI MNYSVSAGLVVGIFIGFQKKAYTSPENLPALVALLLL YGWAVIPMMYPASFLFDVPSTAYVALSCANLFIGINS SAITFILELFENNRTLLRFNAVLRKLLIVFPHFCLGR GLIDLALSQAVTDVYARFGEEHSANPFHWDLIGKNLF AMVVEGVVYFLLTLLVQRHFFLSQWIAEPTKEPIVDE DDDVAEERQRIITGGNKTDILRLHELTKIYPGTSSPA VDRLCVGVRPGECFGLLGVNGAGKTTTFKMLTGDTTV | 11 |
| | TSGDATVAGKSILTNISEVHQNMGYCPQFDAIDELLT GREHLYLYARLRGVPAEEIEKVANWSIKSLGLTVYAD CLAGTYSGGNKRKLSTAIALIGCPPLVLLDEPTTGMD PQARRMLWNVIVSIIREGRAVVLTSHSMEECEALCTR LAIMVKGAFRCMGTIQHLKSKFGDGYIVTMKIKSPKD DLLPDLNPVEQFFQGNFPGSVQRERHYNMLQFQVSSS SLARIFQLLLSHKDSLLIEEYSVTQTTLDQVFVNFAK QQTESHDLPLHPRAAGASRQAQDDYKDHDGDYKDHDI DYKDDDDK | |
| **ABCA4-1188-Npu^{N}**-**3FT** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCFGTGLYLTLVRKMKNIQSQRKGSEGTCSCSSKGF STTCLSYETEILTVEYGLLPIGKIVEKRIECTVYSVD NNGNIYTQPVAQWHDRGEQEVFEYCLEDGSLIRATKD HKFMTVDGQMLPIDEIFERELDLMRVDNLPNDYKDHD GDYKDHDIDYKDDDDK | 12 |
| **ABCA4-1188-Npu^{C}**-**3FT** | MIKIATRKYLGKQNVYDIGVERDHNFALKNGFIASNC PAHVDDLTPEQVLDGDVNELMDVVLHHVPEAKLVECI GQELIFLLPNKNFKHRAYASLFRELEETLADLGLSSF GISDTPLEEIFLKVTEDSDSGPLFAGGAQQKRENVNP RHPCLGPREKAGQTPQDSNVCSPGAPAAHPEGQPPPE PECPGPQLNTGTQLVLQHVQALLVKRFQHTIRSHKDF LAQIVLPATFVFLALMLSIVIPPFGEYPALTLHPWIY GQQYTFFSMDEPGSEQFTVLADVLLNKPGFGNRCLKE GWLPEYPCGNSTPWKTPSVSPNITQLFQKQKWTQVNP SPSCRCSTREKLTMLPECPEGAGGLPPPQRTQRSTEI LQDLTDRNISDFLVKTYPALIRSSLKSKFWVNEQRYG GISIGGKLPVVPITGEALVGFLSDLGRIMNVSGGPIT | 13 |
| | REASKEIPDFLKHLETEDNIKVWFNNKGWHALVSFLN VAHNAILRASLPKDRSPEEYGITVISQPLNLTKEQLS EITVLTTSVDAVVAICVIFSMSFVPASFVLYLIQERV NKSKHLQFISGVSPTTYWVTNFLWDIMNYSVSAGLVV GIFIGFQKKAYTSPENLPALVALLLLYGWAVIPMMYP ASFLFDVPSTAYVALSCANLFIGINSSAITFILELFE NNRTLLRFNAVLRKLLIVFPHFCLGRGLIDLALSQAV TDVYARFGEEHSANPFHWDLIGKNLFAMVVEGVVYFL LTLLVQRHFFLSQWIAEPTKEPIVDEDDDVAEERQRI ITGGNKTDILRLHELTKIYPGTSSPAVDRLCVGVRPG ECFGLLGVNGAGKTTTFKMLTGDTTVTSGDATVAGKS ILTNISEVHQNMGYCPQFDAIDELLTGREHLYLYARL RGVPAEEIEKVANWSIKSLGLTVYADCLAGTYSGGNK RKLSTAIALIGCPPLVLLDEPTTGMDPQARRMLWNVI VSIIREGRAVVLTSHSMEECEALCTRLAIMVKGAFRC MGTIQHLKSKFGDGYIVTMKIKSPKDDLLPDLNPVEQ FFQGNFPGSVQRERHYNMLQFQVSSSSLARIFQLLLS HKDSLLIEEYSVTQTTLDQVFVNFAKQQTESHDLPLH PRAAGASRQAQDDYKDHDGDYKDHDIDYKDDDDK | |
| **EGFP-71-Cfa^{N}** | MVSKGEELFTGVVPILVELDGDVNGHKFSVSGEGEGD ATYGKLTLKFICTTGKLPVPWPTLVTTLTYGVQCLSY DTEILTVEYGFLPIGKIVEERIECTVYTVDKNGFVYT QPIAQWHNRGEQEVFEYCLEDGSIIRATKDHKFMTTD GQMLPIDEIFERGLDLKQVDGLPGHHHHHHG | 14 |
| **EGFP-71-Cfa^{C}** | MVKIISRKSLGTQNVYDIGVEKDHNFLLKNGLVASNC FSRYPDHMKQHDFFKSAMPEGYVQERTIFFKDDGNYK TRAEVKFEGDTLVNRIELKGIDFKEDGNILGHKLEYN YNSHNVYIMADKQKNGIKVNFKIRHNIEDGSVQLADH YQQNTPIGDGPVLLPDNHYLSTQSALSKDPNEKRDHM VLLEFVTAAGITLGMDELYKGHHHHHHG | 15 |
| **EGFP** | MVSKGEELFTGVVPILVELDGDVNGHKFSVSGEGEGD ATYGKLTLKFICTTGKLPVPWPTLVTTLTYGVQCFSR YPDHMKQHDFFKSAMPEGYVQERTIFFKDDGNYKTRA EVKFEGDTLVNRIELKGIDFKEDGNILGHKLEYNYNS HNVYIMADKQKNGIKVNFKIRHNIEDGSVQLADHYQQ NTPIGDGPVLLPDNHYLSTQSALSKDPNEKRDHMVLL EFVTAAGITLGMDELYKGHHHHHHG | 16 |
| **EGFP-71-Npu^{N}** | MVSKGEELFTGVVPILVELDGDVNGHKFSVSGEGEGD ATYGKLTLKFICTTGKLPVPWPTLVTTLTYGVQCLSY ETEILTVEYGLLPIGKIVEKRIECTVYSVDNNGNIYT QPVAQWHDRGEQEVFEYCLEDGSLIRATKDHKFMTVD GQMLPIDEIFERELDLMRVDNLPNGHHHHHHG | 17 |
| **EGFP-71-Npu^{C}** | MIKIATRKYLGKQNVYDIGVERDHNFALKNGFIASNC FSRYPDHMKQHDFFKSAMPEGYVQERTIFFKDDGNYK TRAEVKFEGDTLVNRIELKGIDFKEDGNILGHKLEYN YNSHNVYIMADKQKNGIKVNFKIRHNIEDGSVQLADH YQQNTPIGDGPVLLPDNHYLSTQSALSKDPNEKRDHM VLLEFVTAAGITLGMDELYKGHHHHHHG | 18 |
| **ABCA4-1150-Cfa^{N}**-**3FT-SopE** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR | 19 |
| | VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYDTEILTVEYGFLPIGKIVEERIECTVYTVDK NGFVYTQPIAQWHNRGEQEVFEYCLEDGSIIRATKDH KFMTTDGQMLPIDEIFERGLDLKQVDGLPDYKDHDGD YKDHDIDYKDDDDKTKITLSPQNFRIQKQETTLLKEK STEKNSLAKSILAVKNHFIELRSKLSERFISHKNTES SATHFHRGSASEGRAVLTNKVVKDFMLQTLNDIDIRG SA | |
| **ABCA4-1150-Cfa^{C}**-**3FT-SopE** | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSAVKIISRKSLGT QNVYDIGVEKDHNFLLKNGLVASNCFGTGLYLTLVRK MKNIQSQRKGSEGTCSCSSKGFSTTCPAHVDDLTPEQ VLDGDVNELMDVVLHHVPEAKLVECIGQELIFLLPNK NFKHRAYASLFRELEETLADLGLSSFGISDTPLEEIF LKVTEDSDSGPLFAGGAQQKRENVNPRHPCLGPREKA GQTPQDSNVCSPGAPAAHPEGQPPPEPECPGPQLNTG TQLVLQHVQALLVKRFQHTIRSHKDFLAQIVLPATFV FLALMLSIVIPPFGEYPALTLHPWIYGQQYTFFSMDE PGSEQFTVLADVLLNKPGFGNRCLKEGWLPEYPCGNS TPWKTPSVSPNITQLFQKQKWTQVNPSPSCRCSTREK LTMLPECPEGAGGLPPPQRTQRSTEILQDLTDRNISD FLVKTYPALIRSSLKSKFWVNEQRYGGISIGGKLPVV PITGEALVGFLSDLGRIMNVSGGPITREASKEIPDFL KHLETEDNIKVWFNNKGWHALVSFLNVAHNAILRASL PKDRSPEEYGITVISQPLNLTKEQLSEITVLTTSVDA VVAICVIFSMSFVPASFVLYLIQERVNKSKHLQFISG VSPTTYWVTNFLWDIMNYSVSAGLVVGIFIGFQKKAY TSPENLPALVALLLLYGWAVIPMMYPASFLFDVPSTA YVALSCANLFIGINSSAITFILELFENNRTLLRFNAV LRKLLIVFPHFCLGRGLIDLALSQAVTDVYARFGEEH SANPFHWDLIGKNLFAMVVEGVVYFLLTLLVQRHFFL SQWIAEPTKEPIVDEDDDVAEERQRIITGGNKTDILR LHELTKIYPGTSSPAVDRLCVGVRPGECFGLLGVNGA GKTTTFKMLTGDTTVTSGDATVAGKSILTNISEVHQN MGYCPQFDAIDELLTGREHLYLYARLRGVPAEEIEKV | 20 |
| | ANWSIKSLGLTVYADCLAGTYSGGNKRKLSTAIALIG CPPLVLLDEPTTGMDPQARRMLWNVIVSIIREGRAVV LTSHSMEECEALCTRLAIMVKGAFRCMGTIQHLKSKF GDGYIVTMKIKSPKDDLLPDLNPVEQFFQGNFPGSVQ RERHYNMLQFQVSSSSLARIFQLLLSHKDSLLIEEYS VTQTTLDQVFVNFAKQQTESHDLPLHPRAAGASRQAQ DDYKDHDGDYKDHDIDYKDDDDK | |
| **ABCA4-1140-Cfa^{N}**-**3FT-SopE** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCLSYDTEI LTVEYGFLPIGKIVEERIECTVYTVDKNGFVYTQPIA QWHNRGEQEVFEYCLEDGSIIRATKDHKFMTTDGQML PIDEIFERGLDLKQVDGLPDYKDHDGDYKDHDIDYKD DDDKTKITLSPQNFRIQKQETTLLKEKSTEKNSLAKS ILAVKNHFIELRSKLSERFISHKNTESSATHFHRGSA SEGRAVLTNKVVKDFMLQTLNDIDIRGSA | 21 |
| **ABCA4-1140-Cfa^{C}**-**3FT-SopE** | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSAVKIISRKSLGT QNVYDIGVGEPHNFLLKNGLVASNCSGTPLFLKNCFG TGLYLTLVRKMKNIQSQRKGSEGTCSCSSKGFSTTCP AHVDDLTPEQVLDGDVNELMDVVLHHVPEAKLVECIG QELIFLLPNKNFKHRAYASLFRELEETLADLGLSSFG ISDTPLEEIFLKVTEDSDSGPLFAGGAQQKRENVNPR HPCLGPREKAGQTPQDSNVCSPGAPAAHPEGQPPPEP ECPGPQLNTGTQLVLQHVQALLVKRFQHTIRSHKDFL AQIVLPATFVFLALMLSIVIPPFGEYPALTLHPWIYG QQYTFFSMDEPGSEQFTVLADVLLNKPGFGNRCLKEG WLPEYPCGNSTPWKTPSVSPNITQLFQKQKWTQVNPS | 22 |
| | PSCRCSTREKLTMLPECPEGAGGLPPPQRTQRSTEIL QDLTDRNISDFLVKTYPALIRSSLKSKFWVNEQRYGG ISIGGKLPVVPITGEALVGFLSDLGRIMNVSGGPITR EASKEIPDFLKHLETEDNIKVWFNNKGWHALVSFLNV AHNAILRASLPKDRSPEEYGITVISQPLNLTKEQLSE ITVLTTSVDAVVAICVIFSMSFVPASFVLYLIQERVN KSKHLQFISGVSPTTYWVTNFLWDIMNYSVSAGLVVG IFIGFQKKAYTSPENLPALVALLLLYGWAVIPMMYPA SFLFDVPSTAYVALSCANLFIGINSSAITFILELFEN NRTLLRFNAVLRKLLIVFPHFCLGRGLIDLALSQAVT DVYARFGEEHSANPFHWDLIGKNLFAMVVEGVVYFLL TLLVQRHFFLSQWIAEPTKEPIVDEDDDVAEERQRII TGGNKTDILRLHELTKIYPGTSSPAVDRLCVGVRPGE CFGLLGVNGAGKTTTFKMLTGDTTVTSGDATVAGKSI LTNISEVHQNMGYCPQFDAIDELLTGREHLYLYARLR GVPAEEIEKVANWSIKSLGLTVYADCLAGTYSGGNKR KLSTAIALIGCPPLVLLDEPTTGMDPQARRMLWNVIV SIIREGRAVVLTSHSMEECEALCTRLAIMVKGAFRCM GTIQHLKSKFGDGYIVTMKIKSPKDDLLPDLNPVEQF FQGNFPGSVQRERHYNMLQFQVSSSSLARIFQLLLSH KDSLLIEEYSVTQTTLDQVFVNFAKQQTESHDLPLHP RAAGASRQAQDDYKDHDGDYKDHDIDYKDDDDK | |
| **EGFP-71-Cfa^{N}**-**SopE** | MVSKGEELFTGVVPILVELDGDVNGHKFSVSGEGEGD ATYGKLTLKFICTTGKLPVPWPTLVTTLTYGVQCLSY DTEILTVEYGFLPIGKIVEERIECTVYTVDKNGFVYT QPIAQWHNRGEQEVFEYCLEDGSIIRATKDHKFMTTD GQMLPIDEIFERGLDLKQVDGLPGHHHHHHGTKITLS PQNFRIQKQETTLLKEKSTEKNSLAKSILAVKNHFIE LRSKLSERFISHKNTESSATHFHRGSASEGRAVLTNK VVKDFMLQTLNDIDIRGSA | 23 |
| **EGFP-71-Cfa^{N}**-**SopE** | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSAVKIISRKSLGT QNVYDIGVEKDHNFLLKNGLVASNCFSRYPDHMKQHD FFKSAMPEGYVQERTIFFKDDGNYKTRAEVKFEGDTL VNRIELKGIDFKEDGNILGHKLEYNYNSHNVYIMADK QKNGIKVNFKIRHNIEDGSVQLADHYQQNTPIGDGPV LLPDNHYLSTQSALSKDPNEKRDHMVLLEFVTAAGIT LGMDELYKGHHHHHHG | 24 |
| **EGFP-71-Cfa^{N}-DD1** | MVSKGEELFTGVVPILVELDGDVNGHKFSVSGEGEGD ATYGKLTLKFICTTGKLPVPWPTLVTTLTYGVQCLSY DTEILTVEYGFLPIGKIVEERIECTVYTVDKNGFVYT QPIAQWHNRGEQEVFEYCLEDGSIIRATKDHKFMTTD GQMLPIDEIFERGLDLKQVDGLPGHHHHHHGRISFGS PPPMAGG | 25 |
| **EGFP-71-Cfa^{C}-DD1** | MRISFGSPPPMAGGVKIISRKSLGTQNVYDIGVEKDH NFLLKNGLVASNCFSRYPDHMKQHDFFKSAMPEGYVQ ERTIFFKDDGNYKTRAEVKFEGDTLVNRIELKGIDFK EDGNILGHKLEYNYNSHNVYIMADKQKNGIKVNFKIR HNIEDGSVQLADHYQQNTPIGDGPVLLPDNHYLSTQS ALSKDPNEKRDHMVLLEFVTAAGITLGMDELYKGHHH HHHG | 26 |
| **ABCA4^{N-}1150** | | 60 |
| **ABCA4^{C}**-**1150** | | 61 |
| | | |
| **ABCA4^{N}-1140** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLY | 62 |
| **ABCA4^{C}-1140** | CSGTPLFLKNCFGTGLYLTLVRKMKNIQSQRKGSEGT CSCSSKGFSTTCPAHVDDLTPEQVLDGDVNELMDVVL HHVPEAKLVECIGQELIFLLPNKNFKHRAYASLFREL EETLADLGLSSFGISDTPLEEIFLKVTEDSDSGPLFA GGAQQKRENVNPRHPCLGPREKAGQTPQDSNVCSPGA PAAHPEGQPPPEPECPGPQLNTGTQLVLQHVQALLVK RFQHTIRSHKDFLAQIVLPATFVFLALMLSIVIPPFG EYPALTLHPWIYGQQYTFFSMDEPGSEQFTVLADVLL NKPGFGNRCLKEGWLPEYPCGNSTPWKTPSVSPNITQ LFQKQKWTQVNPSPSCRCSTREKLTMLPECPEGAGGL PPPQRTQRSTEILQDLTDRNISDFLVKTYPALIRSSL KSKFWVNEQRYGGISIGGKLPVVPITGEALVGFLSDL GRIMNVSGGPITREASKEIPDFLKHLETEDNIKVWFN NKGWHALVSFLNVAHNAILRASLPKDRSPEEYGITVI SQPLNLTKEQLSEITVLTTSVDAVVAICVIFSMSFVP ASFVLYLIQERVNKSKHLQFISGVSPTTYWVTNFLWD IMNYSVSAGLVVGIFIGFQKKAYTSPENLPALVALLL LYGWAVIPMMYPASFLFDVPSTAYVALSCANLFIGIN SSAITFILELFENNRTLLRFNAVLRKLLIVFPHFCLG | 63 |
| | RGLIDLALSQAVTDVYARFGEEHSANPFHWDLIGKNL FAMVVEGVVYFLLTLLVQRHFFLSQWIAEPTKEPIVD EDDDVAEERQRIITGGNKTDILRLHELTKIYPGTSSP AVDRLCVGVRPGECFGLLGVNGAGKTTTFKMLTGDTT VTSGDATVAGKSILTNISEVHQNMGYCPQFDAIDELL TGREHLYLYARLRGVPAEEIEKVANWSIKSLGLTVYA DCLAGTYSGGNKRKLSTAIALIGCPPLVLLDEPTTGM DPQARRMLWNVIVSIIREGRAVVLTSHSMEECEALCT RLAIMVKGAFRCMGTIQHLKSKFGDGYIVTMKIKSPK DDLLPDLNPVEQFFQGNFPGSVQRERHYNMLQFQVSS SSLARIFQLLLSHKDSLLIEEYSVTQTTLDQVFVNFA KQQTESHDLPLHPRAAGASRQAQD | |
| **ABCA4^{N}-1188** | | 64 |
| **ABCA4^{C}-1188** | | 65 |
| | | |
| **ABCA4-1150-Cfa^{N}** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYDTEILTVEYGFLPIGKIVEERIECTVYTVDK NGFVYTQPIAQWHNRGEQEVFEYCLEDGSIIRATKDH KFMTTDGQMLPIDEIFERGLDLKQVDGLP | 66 |
| **ABCA4-1150-Cfa^{C}** | MVKIISRKSLGTQNVYDIGVEKDHNFLLKNGLVASNC FGTGLYLTLVRKMKNIQSQRKGSEGTCSCSSKGFSTT CPAHVDDLTPEQVLDGDVNELMDVVLHHVPEAKLVEC IGQELIFLLPNKNFKHRAYASLFRELEETLADLGLSS FGISDTPLEEIFLKVTEDSDSGPLFAGGAQQKRENVN | 67 |
| | PRHPCLGPREKAGQTPQDSNVCSPGAPAAHPEGQPPP EPECPGPQLNTGTQLVLQHVQALLVKRFQHTIRSHKD FLAQIVLPATFVFLALMLSIVIPPFGEYPALTLHPWI YGQQYTFFSMDEPGSEQFTVLADVLLNKPGFGNRCLK EGWLPEYPCGNSTPWKTPSVSPNITQLFQKQKWTQVN PSPSCRCSTREKLTMLPECPEGAGGLPPPQRTQRSTE ILQDLTDRNISDFLVKTYPALIRSSLKSKFWVNEQRY GGISIGGKLPVVPITGEALVGFLSDLGRIMNVSGGPI TREASKEIPDFLKHLETEDNIKVWFNNKGWHALVSFL NVAHNAILRASLPKDRSPEEYGITVISQPLNLTKEQL SEITVLTTSVDAVVAICVIFSMSFVPASFVLYLIQER VNKSKHLQFISGVSPTTYWVTNFLWDIMNYSVSAGLV VGIFIGFQKKAYTSPENLPALVALLLLYGWAVIPMMY PASFLFDVPSTAYVALSCANLFIGINSSAITFILELF ENNRTLLRFNAVLRKLLIVFPHFCLGRGLIDLALSQA VTDVYARFGEEHSANPFHWDLIGKNLFAMVVEGVVYF LLTLLVQRHFFLSQWIAEPTKEPIVDEDDDVAEERQR IITGGNKTDILRLHELTKIYPGTSSPAVDRLCVGVRP GECFGLLGVNGAGKTTTFKMLTGDTTVTSGDATVAGK SILTNISEVHQNMGYCPQFDAIDELLTGREHLYLYAR LRGVPAEEIEKVANWSIKSLGLTVYADCLAGTYSGGN KRKLSTAIALIGCPPLVLLDEPTTGMDPQARRMLWNV IVSIIREGRAVVLTSHSMEECEALCTRLAIMVKGAFR CMGTIQHLKSKFGDGYIVTMKIKSPKDDLLPDLNPVE QFFQGNFPGSVQRERHYNMLQFQVSSSSLARIFQLLL SHKDSLLIEEYSVTQTTLDQVFVNFAKQQTESHDLPL HPRAAGASRQAQD | |
| **ABCA4-1140-Cfa^{N}** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG | 68 |
| | RTIIMSTHHMDEADLLGDRIAIIAQGRLYCLSYDTEI LTVEYGFLPIGKIVEERIECTVYTVDKNGFVYTQPIA QWHNRGEQEVFEYCLEDGSIIRATKDHKFMTTDGQML PIDEIFERGLDLKQVDGLP | |
| **ABCA4-1140-**Cfa^{Cmut} | MVKIISRKSLGTQNVYDIGVGEPHNFLLKNGLVASNC SGTPLFLKNCFGTGLYLTLVRKMKNIQSQRKGSEGTC SCSSKGFSTTCPAHVDDLTPEQVLDGDVNELMDVVLH HVPEAKLVECIGQELIFLLPNKNFKHRAYASLFRELE ETLADLGLSSFGISDTPLEEIFLKVTEDSDSGPLFAG GAQQKRENVNPRHPCLGPREKAGQTPQDSNVCSPGAP AAHPEGQPPPEPECPGPQLNTGTQLVLQHVQALLVKR FQHTIRSHKDFLAQIVLPATFVFLALMLSIVIPPFGE YPALTLHPWIYGQQYTFFSMDEPGSEQFTVLADVLLN KPGFGNRCLKEGWLPEYPCGNSTPWKTPSVSPNITQL FQKQKWTQVNPSPSCRCSTREKLTMLPECPEGAGGLP PPQRTQRSTEILQDLTDRNISDFLVKTYPALIRSSLK SKFWVNEQRYGGISIGGKLPVVPITGEALVGFLSDLG RIMNVSGGPITREASKEIPDFLKHLETEDNIKVWFNN KGWHALVSFLNVAHNAILRASLPKDRSPEEYGITVIS QPLNLTKEQLSEITVLTTSVDAVVAICVIFSMSFVPA SFVLYLIQERVNKSKHLQFISGVSPTTYWVTNFLWDI MNYSVSAGLVVGIFIGFQKKAYTSPENLPALVALLLL YGWAVIPMMYPASFLFDVPSTAYVALSCANLFIGINS SAITFILELFENNRTLLRFNAVLRKLLIVFPHFCLGR GLIDLALSQAVTDVYARFGEEHSANPFHWDLIGKNLF AMVVEGVVYFLLTLLVQRHFFLSQWIAEPTKEPIVDE DDDVAEERQRIITGGNKTDILRLHELTKIYPGTSSPA VDRLCVGVRPGECFGLLGVNGAGKTTTFKMLTGDTTV TSGDATVAGKSILTNISEVHQNMGYCPQFDAIDELLT GREHLYLYARLRGVPAEEIEKVANWSIKSLGLTVYAD CLAGTYSGGNKRKLSTAIALIGCPPLVLLDEPTTGMD PQARRMLWNVIVSIIREGRAVVLTSHSMEECEALCTR LAIMVKGAFRCMGTIQHLKSKFGDGYIVTMKIKSPKD DLLPDLNPVEQFFQGNFPGSVQRERHYNMLQFQVSSS SLARIFQLLLSHKDSLLIEEYSVTQTTLDQVFVNFAK QQTESHDLPLHPRAAGASRQAQD | 69 |
| **ABCA4-1188-Cfa^{N}** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI | 70 |
| | LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCFGTGLYLTLVRKMKNIQSQRKGSEGTCSCSSKGF STTCLSYDTEILTVEYGFLPIGKIVEERIECTVYTVD KNGFVYTQPIAQWHNRGEQEVFEYCLEDGSIIRATKD HKFMTTDGQMLPIDEIFERGLDLKQVDGLP | |
| **ABCA4-1188-Cfa^{cmut}** | MVKIISRKSLGTQNVYDIGVGEPHNFLLKNGLVASNC PAHVDDLTPEQVLDGDVNELMDVVLHHVPEAKLVECI GQELIFLLPNKNFKHRAYASLFRELEETLADLGLSSF GISDTPLEEIFLKVTEDSDSGPLFAGGAQQKRENVNP RHPCLGPREKAGQTPQDSNVCSPGAPAAHPEGQPPPE PECPGPQLNTGTQLVLQHVQALLVKRFQHTIRSHKDF LAQIVLPATFVFLALMLSIVIPPFGEYPALTLHPWIY GQQYTFFSMDEPGSEQFTVLADVLLNKPGFGNRCLKE GWLPEYPCGNSTPWKTPSVSPNITQLFQKQKWTQVNP SPSCRCSTREKLTMLPECPEGAGGLPPPQRTQRSTEI LQDLTDRNISDFLVKTYPALIRSSLKSKFWVNEQRYG GISIGGKLPVVPITGEALVGFLSDLGRIMNVSGGPIT REASKEIPDFLKHLETEDNIKVWFNNKGWHALVSFLN VAHNAILRASLPKDRSPEEYGITVISQPLNLTKEQLS EITVLTTSVDAVVAICVIFSMSFVPASFVLYLIQERV NKSKHLQFISGVSPTTYWVTNFLWDIMNYSVSAGLVV GIFIGFQKKAYTSPENLPALVALLLLYGWAVIPMMYP ASFLFDVPSTAYVALSCANLFIGINSSAITFILELFE NNRTLLRFNAVLRKLLIVFPHFCLGRGLIDLALSQAV TDVYARFGEEHSANPFHWDLIGKNLFAMVVEGVVYFL LTLLVQRHFFLSQWIAEPTKEPIVDEDDDVAEERQRI ITGGNKTDILRLHELTKIYPGTSSPAVDRLCVGVRPG ECFGLLGVNGAGKTTTFKMLTGDTTVTSGDATVAGKS ILTNISEVHQNMGYCPQFDAIDELLTGREHLYLYARL RGVPAEEIEKVANWSIKSLGLTVYADCLAGTYSGGNK RKLSTAIALIGCPPLVLLDEPTTGMDPQARRMLWNVI VSIIREGRAVVLTSHSMEECEALCTRLAIMVKGAFRC MGTIQHLKSKFGDGYIVTMKIKSPKDDLLPDLNPVEQ FFQGNFPGSVQRERHYNMLQFQVSSSSLARIFQLLLS HKDSLLIEEYSVTQTTLDQVFVNFAKQQTESHDLPLH PRAAGASRQAQD | 71 |
| **ABCA4-1150-Cfa^{N}**-**SopE** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN | 72 |
| | STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYDTEILTVEYGFLPIGKIVEERIECTVYTVDK NGFVYTQPIAQWHNRGEQEVFEYCLEDGSIIRATKDH KFMTTDGQMLPIDEIFERGLDLKQVDGLPTKITLSPQ NFRIQKQETTLLKEKSTEKNSLAKSILAVKNHFIELR SKLSERFISHKNTESSATHFHRGSASEGRAVLTNKVV KDFMLQTLNDIDIRGSA | |
| **ABCA4-1150-Cfa^{C}**-**SopE** | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSAVKIISRKSLGT QNVYDIGVEKDHNFLLKNGLVASNCFGTGLYLTLVRK MKNIQSQRKGSEGTCSCSSKGFSTTCPAHVDDLTPEQ VLDGDVNELMDVVLHHVPEAKLVECIGQELIFLLPNK NFKHRAYASLFRELEETLADLGLSSFGISDTPLEEIF LKVTEDSDSGPLFAGGAQQKRENVNPRHPCLGPREKA GQTPQDSNVCSPGAPAAHPEGQPPPEPECPGPQLNTG TQLVLQHVQALLVKRFQHTIRSHKDFLAQIVLPATFV FLALMLSIVIPPFGEYPALTLHPWIYGQQYTFFSMDE PGSEQFTVLADVLLNKPGFGNRCLKEGWLPEYPCGNS TPWKTPSVSPNITQLFQKQKWTQVNPSPSCRCSTREK LTMLPECPEGAGGLPPPQRTQRSTEILQDLTDRNISD FLVKTYPALIRSSLKSKFWVNEQRYGGISIGGKLPVV PITGEALVGFLSDLGRIMNVSGGPITREASKEIPDFL KHLETEDNIKVWFNNKGWHALVSFLNVAHNAILRASL PKDRSPEEYGITVISQPLNLTKEQLSEITVLTTSVDA VVAICVIFSMSFVPASFVLYLIQERVNKSKHLQFISG VSPTTYWVTNFLWDIMNYSVSAGLVVGIFIGFQKKAY TSPENLPALVALLLLYGWAVIPMMYPASFLFDVPSTA YVALSCANLFIGINSSAITFILELFENNRTLLRFNAV LRKLLIVFPHFCLGRGLIDLALSQAVTDVYARFGEEH SANPFHWDLIGKNLFAMVVEGVVYFLLTLLVQRHFFL SQWIAEPTKEPIVDEDDDVAEERQRIITGGNKTDILR LHELTKIYPGTSSPAVDRLCVGVRPGECFGLLGVNGA GKTTTFKMLTGDTTVTSGDATVAGKSILTNISEVHQN MGYCPQFDAIDELLTGREHLYLYARLRGVPAEEIEKV ANWSIKSLGLTVYADCLAGTYSGGNKRKLSTAIALIG CPPLVLLDEPTTGMDPQARRMLWNVIVSIIREGRAVV LTSHSMEECEALCTRLAIMVKGAFRCMGTIQHLKSKF GDGYIVTMKIKSPKDDLLPDLNPVEQFFQGNFPGSVQ | 73 |
| | RERHYNMLQFQVSSSSLARIFQLLLSHKDSLLIEEYS VTQTTLDQVFVNFAKQQTESHDLPLHPRAAGASRQAQ D | |
| **ABCA4-1140-Cfa^{N}**-**SopE** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCLSYDTEI LTVEYGFLPIGKIVEERIECTVYTVDKNGFVYTQPIA QWHNRGEQEVFEYCLEDGSIIRATKDHKFMTTDGQML PIDEIFERGLDLKQVDGLPTKITLSPQNFRIQKQETT LLKEKSTEKNSLAKSILAVKNHFIELRSKLSERFISH KNTESSATHFHRGSASEGRAVLTNKVVKDFMLQTLND IDIRGSA | 74 |
| **ABCA4-1140-Cfa^{Cmut}-SopE** | MTKITLSPQNFRIQKQETTLLKEKSTEKNSLAKSILA VKNHFIELRSKLSERFISHKNTESSATHFHRGSASEG RAVLTNKVVKDFMLQTLNDIDIRGSAVKIISRKSLGT QNVYDIGVGEPHNFLLKNGLVASNCSGTPLFLKNCFG TGLYLTLVRKMKNIQSQRKGSEGTCSCSSKGFSTTCP AHVDDLTPEQVLDGDVNELMDVVLHHVPEAKLVECIG QELIFLLPNKNFKHRAYASLFRELEETLADLGLSSFG ISDTPLEEIFLKVTEDSDSGPLFAGGAQQKRENVNPR HPCLGPREKAGQTPQDSNVCSPGAPAAHPEGQPPPEP ECPGPQLNTGTQLVLQHVQALLVKRFQHTIRSHKDFL AQIVLPATFVFLALMLSIVIPPFGEYPALTLHPWIYG QQYTFFSMDEPGSEQFTVLADVLLNKPGFGNRCLKEG WLPEYPCGNSTPWKTPSVSPNITQLFQKQKWTQVNPS PSCRCSTREKLTMLPECPEGAGGLPPPQRTQRSTEIL QDLTDRNISDFLVKTYPALIRSSLKSKFWVNEQRYGG ISIGGKLPVVPITGEALVGFLSDLGRIMNVSGGPITR EASKEIPDFLKHLETEDNIKVWFNNKGWHALVSFLNV | 75 |
| | AHNAILRASLPKDRSPEEYGITVISQPLNLTKEQLSE ITVLTTSVDAVVAICVIFSMSFVPASFVLYLIQERVN KSKHLQFISGVSPTTYWVTNFLWDIMNYSVSAGLVVG IFIGFQKKAYTSPENLPALVALLLLYGWAVIPMMYPA SFLFDVPSTAYVALSCANLFIGINSSAITFILELFEN NRTLLRFNAVLRKLLIVFPHFCLGRGLIDLALSQAVT DVYARFGEEHSANPFHWDLIGKNLFAMVVEGVVYFLL TLLVQRHFFLSQWIAEPTKEPIVDEDDDVAEERQRII TGGNKTDILRLHELTKIYPGTSSPAVDRLCVGVRPGE CFGLLGVNGAGKTTTFKMLTGDTTVTSGDATVAGKSI LTNISEVHQNMGYCPQFDAIDELLTGREHLYLYARLR GVPAEEIEKVANWSIKSLGLTVYADCLAGTYSGGNKR KLSTAIALIGCPPLVLLDEPTTGMDPQARRMLWNVIV SIIREGRAVVLTSHSMEECEALCTRLAIMVKGAFRCM GTIQHLKSKFGDGYIVTMKIKSPKDDLLPDLNPVEQF FQGNFPGSVQRERHYNMLQFQVSSSSLARIFQLLLSH KDSLLIEEYSVTQTTLDQVFVNFAKQQTESHDLPLHP RAAGASRQAQD | |
| **ABCA4-1150-Cfa^{N}**-**DD1** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA IAFVGDAKVVILDEPTSGVDPYSRRSIWDLLLKYRSG RTIIMSTHHMDEADLLGDRIAIIAQGRLYCSGTPLFL KNCLSYDTEILTVEYGFLPIGKIVEERIECTVYTVDK NGFVYTQPIAQWHNRGEQEVFEYCLEDGSIIRATKDH KFMTTDGQMLPIDEIFERGLDLKQVDGLPRISFGSPP PMAGG | 76 |
| **ABCA4-1150-Cfa^{C}**-**DD1** | MRISFGSPPPMAGGVKIISRKSLGTQNVYDIGVEKDH NFLLKNGLVASNCFGTGLYLTLVRKMKNIQSQRKGSE GTCSCSSKGFSTTCPAHVDDLTPEQVLDGDVNELMDV VLHHVPEAKLVECIGQELIFLLPNKNFKHRAYASLFR | 77 |
| | ELEETLADLGLSSFGISDTPLEEIFLKVTEDSDSGPL FAGGAQQKRENVNPRHPCLGPREKAGQTPQDSNVCSP GAPAAHPEGQPPPEPECPGPQLNTGTQLVLQHVQALL VKRFQHTIRSHKDFLAQIVLPATFVFLALMLSIVIPP FGEYPALTLHPWIYGQQYTFFSMDEPGSEQFTVLADV LLNKPGFGNRCLKEGWLPEYPCGNSTPWKTPSVSPNI TQLFQKQKWTQVNPSPSCRCSTREKLTMLPECPEGAG GLPPPQRTQRSTEILQDLTDRNISDFLVKTYPALIRS SLKSKFWVNEQRYGGISIGGKLPVVPITGEALVGFLS DLGRIMNVSGGPITREASKEIPDFLKHLETEDNIKVW FNNKGWHALVSFLNVAHNAILRASLPKDRSPEEYGIT VISQPLNLTKEQLSEITVLTTSVDAVVAICVIFSMSF VPASFVLYLIQERVNKSKHLQFISGVSPTTYWVTNFL WDIMNYSVSAGLVVGIFIGFQKKAYTSPENLPALVAL LLLYGWAVIPMMYPASFLFDVPSTAYVALSCANLFIG INSSAITFILELFENNRTLLRFNAVLRKLLIVFPHFC LGRGLIDLALSQAVTDVYARFGEEHSANPFHWDLIGK NLFAMVVEGVVYFLLTLLVQRHFFLSQWIAEPTKEPI VDEDDDVAEERQRIITGGNKTDILRLHELTKIYPGTS SPAVDRLCVGVRPGECFGLLGVNGAGKTTTFKMLTGD TTVTSGDATVAGKSILTNISEVHQNMGYCPQFDAIDE LLTGREHLYLYARLRGVPAEEIEKVANWSIKSLGLTV YADCLAGTYSGGNKRKLSTAIALIGCPPLVLLDEPTT GMDPQARRMLWNVIVSIIREGRAVVLTSHSMEECEAL CTRLAIMVKGAFRCMGTIQHLKSKFGDGYIVTMKIKS PKDDLLPDLNPVEQFFQGNFPGSVQRERHYNMLQFQV SSSSLARIFQLLLSHKDSLLIEEYSVTQTTLDQVFVN FAKQQTESHDLPLHPRAAGASRQAQD | |
| **ABCA4-1140-Cfa^{N}**-**DD1** | MGFVRQIQLLLWKNWTLRKRQKIRFVVELVWPLSLFL VLIWLRNANPLYSHHECHFPNKAMPSAGMLPWLQGIF CNVNNPCFQSPTPGESPGIVSNYNNSILARVYRDFQE LLMNAPESQHLGRIWTELHILSQFMDTLRTHPERIAG RGIRIRDILKDEETLTLFLIKNIGLSDSVVYLLINSQ VRPEQFAHGVPDLALKDIACSEALLERFIIFSQRRGA KTVRYALCSLSQGTLQWIEDTLYANVDFFKLFRVLPT LLDSRSQGINLRSWGGILSDMSPRIQEFIHRPSMQDL LWVTRPLMQNGGPETFTKLMGILSDLLCGYPEGGGSR VLSFNWYEDNNYKAFLGIDSTRKDPIYSYDRRTTSFC NALIQSLESNPLTKIAWRAAKPLLMGKILYTPDSPAA RRILKNANSTFEELEHVRKLVKAWEEVGPQIWYFFDN STQMNMIRDTLGNPTVKDFLNRQLGEEGITAEAILNF LYKGPRESQADDMANFDWRDIFNITDRTLRLVNQYLE CLVLDKFESYNDETQLTQRALSLLEENMFWAGVVFPD MYPWTSSLPPHVKYKIRMDIDVVEKTNKIKDRYWDSG PRADPVEDFRYIWGGFAYLQDMVEQGITRSQVQAEAP VGIYLQQMPYPCFVDDSFMIILNRCFPIFMVLAWIYS VSMTVKSIVLEKELRLKETLKNQGVSNAVIWCTWFLD SFSIMSMSIFLLTIFIMHGRILHYSDPFILFLFLLAF STATIMLCFLLSTFFSKASLAAACSGVIYFTLYLPHI LCFAWQDRMTAELKKAVSLLSPVAFGFGTEYLVRFEE QGLGLQWSNIGNSPTEGDEFSFLLSMQMMLLDAAVYG LLAWYLDQVFPGDYGTPLPWYFLLQESYWLGGEGCST REERALEKTEPLTEETEDPEHPEGIHDSFFEREHPGW VPGVCVKNLVKIFEPCGRPAVDRLNITFYENQITAFL GHNGAGKTTTLSILTGLLPPTSGTVLVGGRDIETSLD AVRQSLGMCPQHNILFHHLTVAEHMLFYAQLKGKSQE EAQLEMEAMLEDTGLHHKRNEEAQDLSGGMQRKLSVA | 78 |
| | IAFVGDAKVVILDEPTSGVDPYSRRSIWDLLLKYRSG RTIIMSTHHMDEADLLGDRIAIIAQGRLYCLSYDTEI LTVEYGFLPIGKIVEERIECTVYTVDKNGFVYTQPIA QWHNRGEQEVFEYCLEDGSIIRATKDHKFMTTDGQML PIDEIFERGLDLKQVDGLPRISFGSPPPMAGG | |
| **ABCA4-1140-Cfa^{Cmut}-DD1** | MRISFGSPPPMAGGVKIISRKSLGTQNVYDIGVGEPH NFLLKNGLVASNCSGTPLFLKNCFGTGLYLTLVRKMK NIQSQRKGSEGTCSCSSKGFSTTCPAHVDDLTPEQVL DGDVNELMDVVLHHVPEAKLVECIGQELIFLLPNKNF KHRAYASLFRELEETLADLGLSSFGISDTPLEEIFLK VTEDSDSGPLFAGGAQQKRENVNPRHPCLGPREKAGQ TPQDSNVCSPGAPAAHPEGQPPPEPECPGPQLNTGTQ LVLQHVQALLVKRFQHTIRSHKDFLAQIVLPATFVFL ALMLSIVIPPFGEYPALTLHPWIYGQQYTFFSMDEPG SEQFTVLADVLLNKPGFGNRCLKEGWLPEYPCGNSTP WKTPSVSPNITQLFQKQKWTQVNPSPSCRCSTREKLT MLPECPEGAGGLPPPQRTQRSTEILQDLTDRNISDFL VKTYPALIRSSLKSKFWVNEQRYGGISIGGKLPVVPI TGEALVGFLSDLGRIMNVSGGPITREASKEIPDFLKH LETEDNIKVWFNNKGWHALVSFLNVAHNAILRASLPK DRSPEEYGITVISQPLNLTKEQLSEITVLTTSVDAVV AICVIFSMSFVPASFVLYLIQERVNKSKHLQFISGVS PTTYWVTNFLWDIMNYSVSAGLVVGIFIGFQKKAYTS PENLPALVALLLLYGWAVIPMMYPASFLFDVPSTAYV ALSCANLFIGINSSAITFILELFENNRTLLRFNAVLR KLLIVFPHFCLGRGLIDLALSQAVTDVYARFGEEHSA NPFHWDLIGKNLFAMVVEGVVYFLLTLLVQRHFFLSQ WIAEPTKEPIVDEDDDVAEERQRIITGGNKTDILRLH ELTKIYPGTSSPAVDRLCVGVRPGECFGLLGVNGAGK TTTFKMLTGDTTVTSGDATVAGKSILTNISEVHQNMG YCPQFDAIDELLTGREHLYLYARLRGVPAEEIEKVAN WSIKSLGLTVYADCLAGTYSGGNKRKLSTAIALIGCP PLVLLDEPTTGMDPQARRMLWNVIVSIIREGRAVVLT SHSMEECEALCTRLAIMVKGAFRCMGTIQHLKSKFGD GYIVTMKIKSPKDDLLPDLNPVEQFFQGNFPGSVQRE RHYNMLQFQVSSSSLARIFQLLLSHKDSLLIEEYSVT QTTLDQVFVNFAKQQTESHDLPLHPRAAGASRQAQD | 79 |

### Materials and Methods

### Materials:

Oligonucleotides were purchased from Eurofins genomics. Synthetic genes were purchased from GENEWIZ. Pfu Ultra fusion polymerase for cloning and all restriction enzymes were purchased from Thermofisher Scientific. High-competency cells used for cloning were generated from XL10-Gold chemically competent E. coli. HEK293T cells were purchased from ATCC. DNA purification kits were purchased from Qiagen. All plasmids were sequenced by Macrogen. Luria Bertani (LB) media, and all buffering salts were purchased from Thermofisher Scientific. Coomassie brilliant blue, MG-132 proteosome inhibitor, phenylmethane sulfonyl fluoride, iodoacetamide, NH4HC03, DTT, formic acid, fetal bovine serum and asolectin from soybean were purchased from Sigma-Aldrich. Acetonitrile (ACN) was purchased from Carlo-Erba. EDTA-free complete protease inhibitors were purchased from Roche. Lipofectamine 2000 transfection reagent, DMEM high glucose GlutaMAX supplement, RPMI 1640 medium GlutaMAX supplement, RIPA lysis and extraction buffer, BCA protein assay kit, MES-SDS running buffer, pre-stained protein ladder and SDS-PAGE (Bis-tris and Tris-acetate gels) were purchased from Thermofisher Scientific. The primary anti-6xHis tag mouse monoclonal antibody, anti-flag tag mouse monoclonal antibody, anti-ABCA4 rabbit polyclonal antibody and anti-tubulin rabbit polyclonal antibody were purchased from Invitrogen. The secondary goat anti-mouse IgG (H+L) highly cross-adsorbed antibody alexa fluor plus 680 and goat anti-rabbit IgG (H+L) secondary antibody dylight 800 4X PEG were purchased from Invitrogen. Dodecyl maltoside (D310) and cholesteryl hemisuccinate (CH210) solution were purchased from Anatrace. Trypsin was purchased from Promega.

### Equipment:

Electrospray ionization mass spectrometric analysis (ESI-MS) was carried out on a no Acquity liquid chromatographer (Waters) coupled to a LTQ-Orbitrap Velos (Thermo Scientific) mass spectrometer. Gels and Western-blots were imaged with a LI-COR Odyssey Infrared Imager. Cell lysis was carried out using a SFX550 Branson sonifier. FACS measurements were performed on a Gallios Beckman Coulter.

### Cloning of Recombinant DNA

Synthetic genes to prepare constructs ABCA4-1150-CfaN (SEQ ID NO 1) and ABCA4-1150-CfaC (SEQ ID NO 2) were purchased and introduced into pEGFP-N1 expression vectors using KpnI and NotI restriction enzymes. Synthetic genes for NpuN and NpuC were purchased and introduced into ABCA4-1150-CfaN and ABCA4-1150-CfaC by restriction enzyme free cloning to obtain the constructs ABCA4-1150-NpuN (SEQ ID NO 8) and ABCA4-1150-NpuC (SEQ ID NO 9).

Constructs ABCA4-3FT (SEQ ID NO 7), ABCA4-1140-CfaN (SEQ ID NO 3), ABCA4-1140-CfaCmut (SEQ ID NO 4), ABCA4-1188-CfaN (SEQ ID NO 5) and ABCA4-1188-CfaCmut (SEQ ID NO 6) were prepared by restriction enzyme free cloning. The mutations at the CfaCmut were introduced using inverse PCR with Pfu Ultra II HF Polymerase. ABCA4-1140-NpuN (SEQ ID NO 10), ABCA4-1140-NpuC (SEQ ID NO 11), ABCA4-1188-NpuN (SEQ ID NO 12) and ABCA4-1188-NpuC (SEQ ID NO 13) were prepared by restriction enzyme free cloning.

Construct EGFP-71- CfaN (SEQ ID NO 14), EGFP-71-CfaC (SEQ ID NO 15), EGFP (SEQ ID NO 16), EGFP-71- NpuN (SEQ ID NO 17) and EGFP-71-NpuC (SEQ ID NO 18) were prepared by restriction enzyme free cloning.

Synthetic gene for SopE was purchased and introduced into ABCA4-1150-CfaN, ABCA4-1150-CfaC, ABCA4-1140-CfaN, ABCA4-1140-CfaC, EGFP-71-CfaN and EGFP-71-CfaC by restriction enzyme free cloning to obtain the constructs ABCA4-1150-CfaN-SopE (SEQ ID NO 19), ABCA4-1150-CfaC-SopE (SEQ ID NO 20), ABCA4-1140-CfaN-SopE (SEQ ID NO 21), ABCA4-1140-CfaC-SopE (SEQ ID NO 22), EGFP-71-CfaN-SopE (SEQ ID NO 23) and EGFP-71-CfaC-SopE (SEQ ID NO 24). The gene for DD1 was prepared by overlapped extension PCR and introduced into EGFP-71-CfaN and EGFP-71-CfaC by restriction enzyme free cloning to obtain the constructs EGFP-71-CfaN-DD1 (SEQ ID NO 25) and EGFP-71-CfaC-DD1 (SEQ ID NO 26). The identity of all recombinant plasmids was confirmed through sequencing and the corresponding protein sequences are reported in Table 1.

### Transfection of intein plasmids in HEK293 cells:

HEK293T cells were maintained in DMEM with 10% FBS and antibiotics at 37°C in a 6% CO2 atmosphere. Cells were co-transfected at around 80% confluence using Lipofectamine 2000 and 1.25 µg of each plasmid in 6-well plate format. For the experiments where the plasmid encoded the full-length gene was used, a scramble plasmid was co-transfected with the full-length plasmid to achieve the same amount of DNA transfected when two intein plasmid were used. Cells were harvested after 48 h post-transfection and EGFP was analyzed by Western blot.

### Transfection of degron-intein plasmids in HEK293 cells:

Cells were co-transfected at around 80% confluence using Lipofectamine 2000 and 1.25 µg of each plasmid in 6-well plate format. Cells transfected with intein-degron plasmids were harvested after 48 h post-transfection and analyzed by Western blot. For time course experiments, cells were harvested after 24 and 48 h post-transfection and analyzed by Western blot.

Proteosome inhibitor experiments were performed using MG-132 inhibitor. Cells were co-transfected and 24 h post-transfection DMSO or MG-132 (dissolved in DMSO) was added to a final concentration of 50 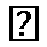 M. Cells were harvested after 30 min, 3, 6 and 24 hours and analyzed by Western blot.

### Western blot analysis:

EGFP transfected cells (HEK293 and WERI-RB1 cells) were lysed in RIPA buffer supplemented with protease inhibitors and 1 mM phenylmethylsulfonyl. After lysis, samples were quantified by BCA protein assay kit. Samples with 10 µg of total protein were denatured at 95°C for 10 minutes in 1X Laemmli sample buffer. Lysates were separated by 12% Bis-tris SDS-PAGE gels for 40 min at 165V. The antibodies used for immuno-blotting were anti-6xHis tag to detect the EGFP and anti-β-tubulin as loading control. The quantification of EGFP bands detected by Western blot was performed using LI-COR Odyssey Infrared Imager.

ABCA4 transfected cells (HEK293) were lysed in dodecyl maltoside (D310) and cholesteryl hemisuccinate (CH210) solution in PBS (1:1) supplemented with protease inhibitors and 1 mM phenylmethylsulfonyl. After lysis, ABCA4 samples were quantified by BCA protein assay kit. Samples with 25 µg of total protein were denatured at 37°C for 15 minutes in 1X Laemmli sample buffer containing 2.5 mg/ml of asolectin. Lysates were separated by 3-8% Tris-acetate SDS-PAGE gels for 1.5 h at 150V. The antibodies used for immuno-blotting were either anti-flag tag or anti-ABCA4 to detect the ABCA4 protein and anti-β-tubulin as loading control. The quantification of ABCA4 bands detected by Western blot was performed using LI-COR Odyssey Infrared Imager.

### Fluorescence-activated cell sorting measurement:

HEK293T cells were co-transfected with 2 µg of each EGFP-intein plasmid and as a negative control HEK293T cells were grown but they were not transfected. For the experiments where the plasmid encoded the full-length gene was used, a scramble plasmid was co-transfected with the full-length plasmid to achieve the same amount of DNA transfected when two intein plasmid were used. Cells were analyzed by flow cytometry after 48 h post-transfection. Transfected and untransfected cells were respectively resuspended in FACS analysis buffer (PBS, 2% FSA, 2 mM EDTA). The percentages of EGFP+ cells were assessed by comparing the different transfected cells to un-transfected cells in a Beckman Coulter Gallios flow cytometer using the Kaluza flow cytometry analysis software. Dapi staining was used to detect the number of dead cells.

### Mass spectrometry analysis:

The gel bands were washed with ammonium bicarbonate (50 mM NH4HCO3) and acetonitrile (ACN). The samples were reduced with 20 mM DTT for 60 min at 60 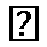 C and alkylated with 55 mM iodoacetamide at 25ºC for 30 min in the dark. Afterwards, the samples were digested double digested for 2 h and overnight at 37ºC with trypsin (sequence grade modified Trypsin). Finally, the resulting peptide mixtures were extracted from the gel matrix with 5% formic acid (FA) in 50% ACN, and 100 % ACN, and dried-down in a SpeedVac vacuum system. The resulting peptide mixtures were cleaned-up with a C18 tip (PolyLC Inc.) as per manufacturer's protocol. Finally, the cleaned-up peptide solutions were dried-down. The tryptic digest mixture was resuspended in 1% FA solution and for each sample, an aliquot was injected for chromatographic separation. Peptides were trapped on a Symmetry C18 trap column (5µm 180µm x 20mm; Waters) and were separated using a C18 reverse phase capillary column (ACQUITY UPLC M-Class Peptide BEH column; 130Å, 1.7µm, 75 µm x 250 mm, Waters). The gradient used for the elution of the peptides was 1 to 40 % B in 25 minutes, followed by gradient from 40% to 60% in 5min (A: 0.1% FA; B: 100% ACN, 0.1%FA), with a 250 nL/min flow rate. Eluted peptides were subjected to electrospray ionization in an emitter needle (PicoTipTM, New Objective) with an applied voltage of 2000V. Peptide masses (m/z 300-1700) were analyzed in data dependent mode where a full Scan MS was acquired in the Orbitrap with a resolution of 60,000 FWHM at 400m/z. Up to the 15th most abundant peptides (minimum intensity of 500 counts) were selected from each MS scan and then fragmented in the linear ion trap using CID (38% normalized collision energy) with helium as the collision gas. Database search was performed with Sequest HT search engine using Thermo Proteome Discover.

### Results and discussion

Recently several new inteins have been engineered based on consensus design ((Stevens et al., 2016; Stevens, Sekar, Gramespacher, Cowburn, & Muir, 2018)) and shown to have superior properties than naturally occurring inteins. One of these inteins, termed Cfa, was generated by consensus design from an alignment of DnaE inteins and shown to have superior properties to some of the best performing inteins of the DnaE family such as Npu. Cfa was reported to have faster kinetics, higher expression levels and high tolerance to extreme conditions such as high temperature and concentration of denaturing agents. Interestingly, Cfa variants with degrees of homology from 90% or higher display similar properties. The same consensus design strategy was applied to the TerL-AceL intein family, which resulted in a consensus sequence termed Cat, which also shows improved properties relative to the rest of the members of the TerL-AceL family.

In order to demonstrate that consensus inteins provided a benefit for the reconstitution of proteins via protein trans-splicing for gene therapy applications, a direct comparison was performed. Comparisons were performed by transfecting cells with purified plasmids.

Initial experiments were performed using EGFP as a reporter gene. Briefly, EGFP was split at position 71 and the N-terminal fragment (residues 1-70, EGFPN) recombinantly fused to N-inteins, and the C-terminal fragment (residues 71-239, EGFPC) to the C-inteins. The following four constructs were generated: ,-CfaN, EGFPN-NpuN, CfaC-EGFPC and NpuC-EGFP, in order to compare the reconstitution efficiency of the Cfa consensus sequence with Npu. Cultured HEK293 cells were co-transfected with equimolar amounts of plasmids encoding for the N and C-terminal fragments and splicing efficiency monitored by fluorescence microscopy, flow cytometry analysis and Western blotting.

Our results, see figures 2, 3 and 4, show that the consensus Cfa intein provides a 2.5 fold increase in EGFP reconstitution compared with the Npu intein, indicating that inteins obtained by consensus design provided higher yields of target protein upon co-transfection. It is important to point out that although Cfa was known to split at faster rates than Npu in vitro, that its N-terminal fragments expressed better when individually transformed or transfected in cells, **it had not been shown if this resulted in a benefit when both fragments were co-transfected in the same cell.** Through these experiments we demonstrate, that when both IntN and IntC fragments are expressed in the same cell, the use of Cfa versus previously known unltra-fast split intein, such as Npu, provides a clear benefit in the reconstitution yield of the target protein.

In order to confirm that this positive feature of the Cfa consensus sequence was observed in other proteins we repeated the experiment using the ABCA4 protein. ABCA4 is a large protein, which is mutated in Stargardt disease, and whose reconstitution has been proposed as a viable strategy to treat the disease. Several approaches based on AAV gene therapy are currently being explored to reconstitute it. Due to its large size ABACA4 cannot be encapsulated into a single AAV, and so different strategies have been proposed to be able to deliver ABCA4 in two fragments, to be reconstituted inside the target cells. In order to confirm that engineered consensus sequences such as Cfa provided a benefit over naturally occurring inteins such as Npu, we split ABCA4 at position 1150 and cloned the N-inteins and C-inteins to the N- and C-terminal fragments of the protein, respectively.

ABCA4^{N}(1-1149)-IntN and IntC-ABCA4^{C}(1150-2273) with the Cfa, or Npu inteins were cloned into expression plasmids and co-transfected into HEK293 cells. Cells were lysed and protein reconstitution yields determined by Western Blot. Full-length ABCA4 was also co-transfected to serve as control.

As observed with EGFP we observed higher reconstitution yields with Cfa than with Npu (see figure 5).

A variant of Cfa, which contains specific mutations in the CfaC fragment, termed CfaCmut ((Stevens et al., 2017)), has also been recently reported to add to the Cfa benefits. Particularly the CfaCmut variant has been shown to be able to efficiently splice protein fragments even if the +2 position of the C-extein does not contain the native Phe residue. The +2 position, as defined above, refers to the position +2, relative to the last amino acid of the IntC. When ABCA4 is split at position 1150, that leaves a Phe in the C-extein +2 position, which is an optimal residue for Npu, as well as Cfa.

Despite ABCA4 can be reconstituted by splitting it at position 1150, this might not be the best possible position. In order to test our ability to reconstitute ABCA4 by splitting it at different sites than 1150, we used the CfaCmut, in order not to be limited to positions that would render a Phe at the position +2, relative to the intein.

Several ABCA4 constructs were cloned in which the ABCA4 protein was split at sites that did not contain a Phe on the position +2 of the split site. Two of the additional sites that were tested where position 1140, which corresponds to a Ser at the +2 site, and position 1188 which corresponds to a Pro at the +2 site. Constructs were cloned and tested as shown above. Specifically, ABCA4(1-1139)-IntN, IntC-ABCA4(1140-2273) constructs were used to evaluate splicing ABCA4 at position 1140. And ABCA4(1-1187)-IntN and IntC-ABCA4(1188-2273) to evaluate splitting ABCA4 at position 1188. Sites were selected based on the topological structure of ABCA4, and taking into consideration the presence of folded domains. Sites were selected outside of well-defined folded domains in the intracellular side of ABCA4. Different sites were tested and it was observed for all of them that Cfa and Cfa variants provided higher reconstitution yields than Npu and that the efficiency was also different depending on the split site. (figure 5, 6 and 7)

Besides reconstitution yield another of the major limitations of the use of split inteins in gene therapy is the presence of unreacted starting materials and/or inteins. To prevent the accumulation of undesired starting materials and to eliminate the excised intein fragments we added a degron to the constructs with the general architecture shown in figure 8.

We have identified several degrons that could be used in combination with inteins to develop gene therapy approaches for ABCA4, which could be translated to diseases caused by mutations on other large genes, as well as to reconstitute proteins to treat those diseases, for example to reconstitute CRISPR/Cas9 systems.

In order to identify suitable degron-intein combinations EGFP-Int constructs were cloned including the selected degron. The degron was cloned at the C-terminus of the N-intein, and the N-terminus of the C-intein. A His6 tag was included as a linker between the two elements for detection purposes. As a proof of principle the SopE destabilizing domain (1-100) was used, as well as a degron consisting of the peptide sequence DD1 (see Table 3)

Results show that the inclusion of the degron indeed removes any detectable amounts of starting materials and inteins (see figure 9, figure 13).

Interestingly, when the degron strategy was applied to the large gene ABCA 4 we observed a similar result but also an increase on the levels of spliced ABCA4 product, indicating an unexpected synergistic effect between consensus inteins and degrons (see figure 10).

The effect on these results of inhibitors of the proteosome was also studied (figure 11). We showed that proteosome inhibitors reduced the effect of the degron, thus confirming that SopE degradation was proteosomally mediated. Interestingly, we observed that combining inteins with degrons we were able not only to eliminate the presence of starting materials but also to increase the level of reconstitution of ABCA4. (figure 10).

We also performed experiments to study the effect of combining consensus intein Cfa with degrons and compared it with the absence of degrons or the use of other inteins. Interestingly, we observed (figure 12) that the use of Cfa with degrons very significantly reduced the levels of starting materials and also increased the amount of product. We also confirmed that this effect was also observed at position 1140 of ABCA4 (figure 13).

Based on the results obtained with ABCA4, other diseases, genes and proteins in which this approach could be applied include the ones shown in table 1.

### Example 2. Three-piece ligation strategy using orthogonal split intein pairs

### Material and methods:

### Transfection of three-piece plasmids in HEK293 cells:

Cells were co-transfected at around 80% confluence using Lipofectamine 2000 and 1.25 µg of each plasmid in 6-well plate format. Cells transfected with three-piece plasmids were harvested after 48 h post-transfection and analyzed by Western blot. As a control, cells were co-transfected with two plasmids (POIN-CfaN+CfaC-POIM-CatN, CfaC-POIM-CatN+CatC-POIC and POIN-CfaN+ CatC-POIC).

### Western blot analysis:

Three-piece transfected cells (HEK293) were lysed in RIPA buffer supplemented with protease inhibitors and 1 mM phenylmethylsulfonyl. After lysis, samples were quantified by BCA protein assay kit. Samples with 10 µg of total protein were denatured at 95°C for 10 minutes in 1X Laemmli sample buffer. Lysates were separated by 12% Bis-tris SDS-PAGE gels for 40 min at 165V. The antibodies used for immuno-blottingwere anti-flag tag to detect the POI and anti-β-tubulin as loading control.

### Results:

We showed combining ultrafast, consensus inteins with degrons is a viable strategy to reconstitute large proteins, while reducing the levels of starting materials and excised inteins. This strategy could be used in gene therapy to reconstitute large proteins in gene replacement strategies, as well as to generate therapeutic agents *in vivo,* when combined with suitable delivery vectors, such as the ones disclosed here. However, for even larger proteins this strategy might not be sufficient. For example, for proteins whose coding region is larger than 7-8 kb, splitting their coding genes in two fragments would not be sufficient to yield fragments that can be encapsulated in AAV vectors. For such proteins, the coding gene may need to be split into three pieces. In order to assemble a protein from three separate fragments using inteins, orthogonal intein pairs would be required. To achieve maximum reconstitution yields, highly efficient, orthogonal inteins would be required. We decided to use Cfa in combination with Cat inteins. These two inteins have been obtained by consensus design and share some common features, including high expression yields, thermostability, tolerance to chaotropic agents and fast splicing kinetics. We designed a series of constructs following the architecture described in Figure 16: POIN-CfaN, CfaC-POIM-CatN and CatC-POIC. Alternatively, the positions of the Cfa and Cat inteins could be swapped, to generate constructs with the following architecture: POIN-CatN, CatC-POIM-CfaN and CfaC-POIC

We tested their orthogonality, and demonstrated that both inteins were indeed orthogonal and they did not react with each other, that is that the N-fragment of the Cfa, or Cat intein, only reacted with their respective cognate pair. When the three fragments were co-transfected in equimolar amounts the main product that was detected was the one arising from the assembly of the three fragments into the full length desired product. Importantly, we only detected low levels of unreacted materials and we did not detect any intermediate product, that is products arising from the reaction of only two of the fragments. This result indicates that the use of highly active pairs of orthogonal consensus inteins is a suitable strategy to assemble large proteins from three individual fragments.

## Claims

1. A composition comprising a first polynucleotide encoding a polynucleotide comprising a Split intein N-fragment directly linked via a peptide bond, optionally through a peptide linker, to the N-terminal fragment of a protein to be reconstituted, and a second polynucleotide encoding a polynucleotide comprising a Split intein C-fragment directly linked via a peptide bond, optionally through a peptide linker, to the C-terminal fragment of the protein to be reconstituted;
wherein both components of the composition may be packed together in a single formulation or separately in different formulations;
wherein the first and the second polynucleotides, respectively, encode the N-terminal fragment and the C-terminal fragment of the protein to be reconstituted, in such a way that when both fragments are combined, the N-terminal fragment of the protein is linked to the C-terminal fragment of the protein generating the whole protein;
wherein each polynucleotide must encode a split intein so that, once translated into a protein, the N-terminal and C-terminal sequences become separate fragments that can non-covalently re-associate, or reconstitute, into an intein that is functional for trans-splicing reactions; and wherein
the protein to be reconstituted is of more than 25 KDa.

2. The composition of claim 2, wherein the first polynucleotide encodes a split intein N-fragment directly linked via a peptide bond to a degron, wherein the degron is linked to the intein N-fragment via the C-terminus of the intein, with or without a linker between the intein N-fragment and the degron, and wherein the N-terminus of the Split intein N-fragment is directly linked via a peptide bond to the N-terminal fragment of the protein to be reconstituted; and wherein the second polynucleotide encodes a split intein C-fragment directly linked via a peptide bond to a degron, wherein the degron is linked to the intein C-fragment via the N-terminus of the intein, with or without a linker between the intein C-fragment and the degron, and wherein the C-terminus of the Split intein C-fragment is directly linked via a peptide bond to the C-terminal fragment of the protein to be reconstituted.

3. The composition of any of claims 1 or 2, wherein the first polynucleotide encodes the CfaN intein of SEQ ID NO 27 or any of its variants, and the second polynucleotide encodes the CfaC intein of SEQ ID NO 28, or any of its variants, wherein variants are understood as split intein N or C-fragments of SEQ ID NO: 27 or SEQ ID NO 28 having at least 90% sequence identity with any of these sequences.

4. The composition of any of claims 2 or 3, wherein degrons are selected from the list consisting of SEQ ID NO 40 to 59, 34, 35 and 37.

5. The composition of claim 2, wherein the split inteins are as defined in claim 3 and wherein the degrons are selected from the list consisting of SEQ ID NO 42, 43, 49, 50, 51, 54 and 34.

6. The composition of any of claims 1 to 5, wherein both polynucleotides are comprised within vectors that allow the propagation of said polynucleotides in suitable host cells.

7. The composition of claim 6, wherein the are adeno associated viruses (AAV).

8. The composition of claim 7, wherein the vectors are AAVs of serotype 1, 2, 3, 4, 5, 6, 7, 8, or 9.

9. The composition of any of claims 1 to 8, wherein the gene coding the whole protein is selected from the list consisting of any of the proteins listed in table 1.

10. The composition as defined in any of claims 1 to 9, for use in therapy.

11. A method for expressing a gene of interest in a cell, which comprises:
(i) contacting the cell with
(a) a first polynucleotide as defined in claim 1, and
(b) a second polynucleotide as defined in claim 1,
(ii) allowing the expression of the first and the second polynucleotides so that the first and the second fusion proteins are produced and
(iii) allowing the contact between the first and second proteins so that the split intein N-fragment binds to the split intein C-fragment to form an intein intermediate and the intein intermediate reacts to covalently link the C-terminus of the first polypeptide of interest to the N-terminus of the second polypeptide of interest.

12. A method for expressing a gene of interest in a cell, which comprises:
(i) contacting the cell with
(a) a first polynucleotide as defined in claim 2, and
(b) a second polynucleotide as defined in claim 2,
(ii) allowing the expression of the first and the second polynucleotides so that the first and the second fusion proteins are produced and
(iii) allowing the contact between the first and second proteins so that the split intein N-fragment binds to the split intein C-fragment to form an intein intermediate and the intein intermediate reacts to covalently link the C-terminus of the first polypeptide of interest to the N-terminus of the second polypeptide of interest.

13. The method of any of claims 11 or 12, wherein the first polynucleotide encodes the CfaN intein of SEQ ID NO 27 or any of its variants, and the second polynucleotide encodes the CfaC intein of SEQ ID NO 28 or any of its variants, wherein variants are understood as split intein N or C-fragments of SEQ ID NO: 27 or SEQ ID NO 28 having at least 90% sequence identity with any of these sequences.

14. The method of any of claims 11 or 12, wherein the split inteins are as defined in claim 3 and wherein the degrons are selected from the list consisting of SEQ ID NO 42, 43, 49, 50, 51, 54 and 34.

15. The method of any of claims 11 to 14, wherein both polynucleotides are comprised within adeno associated viruses (AAV).
